**Europäisches Patentamt**

**European Patent Office**

**Office européen des brevets**

(11) Publication number: **0 359 164**

**A2**

# EUROPEAN PATENT APPLICATION

(21) Application number: **89116734.8**

(51) Int. Cl.5 **C12N 9/60 , C12P 21/06**

(22) Date of filing: **09.09.89**

| | |
|---|---|
| Claims for the following Contracting State: ES. | (71) Applicant: **THE GENERAL HOSPITAL CORPORATION**<br>**55 Fruit Street**<br>**Boston MA 02114(US)** |
| (30) Priority: **13.09.88 US 243733**<br>**14.12.88 US 284244** | |
| (43) Date of publication of application:<br>**21.03.90 Bulletin 90/12** | (72) Inventor: **Smith, John A.**<br>**19 Thatcher Street No. 5**<br>**Brookline Massachusetts 02146(US)**<br>Inventor: **Chang, Yie-Hwa** |
| (84) Designated Contracting States:<br>**AT BE CH DE ES FR GB GR IT LI LU NL SE** | ´ **91 Winslow Avenue**<br>**Somerville Massachusetts 02143(US)** |
| | (74) Representative: **Klein, Otto, Dr. et al**<br>**Hoechst AG Zentrale Patentabteilung**<br>**Postfach 80 03 20**<br>**D-6230 Frankfurt am Main 80(DE)** |

(54) **Isolation, purification, and characterization of the aminopeptidases: AP2, API, and APX.**

(57) This invention is directed toward substrate-specific aminopeptidase enzymes, and specifically toward a substrate-specific aminopeptidase produced via natural or recombinant methods. The invention is further directed to a method for purifying and using the substrate-specific aminopeptidase. The invention also is directed toward a vacuolar localization sequence.

EP 0 359 164 A2

## ISOLATION, PURIFICATION, AND CHARACTERIZATION OF THE AMINOPEPTIDASES: AP2, API, AND APX

### Field of the Invention

The invention is directed to the isolation and purification of substrate-specific aminopeptidases and to the enzymes themselves.

### Background of the Invention

Enzymes which are capable of cleaving a peptide bond between the amino-terminal residue of a protein or peptide and an adjacent amino acid residue are known as "aminopeptidases". Such enzymes have been identified in a variety of microorganisms, such as E. coli, Bacillus licheniformis, Bacillus subtilis, Salmonella typhimurium and Mycoplasma salivarium (Carter, T. H. et al., J. Bacteriol. 159: 453-459 (1984); Hayashi, S., J. Biol. Chem. 259:10448-10454 (1984); Hermsdorf, C. L., Biochem. 17: 3370-3376 (1978); McHugh, G. L. et al., J. Bacteriol. 120: 364-371 (1974); Miller, C. G. et al., J. Bacteriol. 135: 603-611 (1978), Simmonds, S., Biochem. 9:1-8 (1970); and Shibata, K. et al., J. Bacteriol. 169:3409-3413 (1987)).

One example of an aminopeptidase is the leucine-specific aminopeptidase produced in mammalian intestinal mucosa and kidneys. The leucine-specific aminopeptidase of swine intestinal fluid has been purified and characterized, and found to contain a $Zn^{+2}$ ion. The enzyme has a broad substrate specificity with respect to the N-terminal residue of the substrate peptide. By successive hydrolysis of N-terminal peptide bonds, it can degrade peptides to free amino acids. The enzyme has been found to be activated by $Mn^{+2}$, which is capable of replacing the zinc ion (White, A. et al., In: Principles of Biochemistry, 5th Ed., McGraw Hill, NY, NY (1973)). A second example of an aminopeptidase is the methionine-specific aminopeptidase of E. coli (Ben-Bassat et al., J. Bacteriol. 169:751-757 (1987)). A theoretical discussion of the properties of aminopeptidases is provides by Sherman, F. et al. (BioEssays 3:27-31 (1985)).

Yeast, and in particular, the yeast Saccharomyces cerevisiae, have also been found to contain aminopeptidases. Trumbly, R.J. et al. (J. Bacteriol. 156:36-48 (1983)), for example, identified a yeast leucine aminopeptidase which was capable of cleaving a leucine-containing peptide. Yeast peptidases are reviewed by Achstetter, T. et al. (Yeast 1:139-157 (1985)), which reference is herein incorporated by reference. Aminopeptidases are also produced in the human small intestine (Martin, D.W. et al., In: Harper's Review of Biochemistry, 19th Ed., Lange Medical Publications, Los Altos, CA (1983)).

The yeast aminopeptidase, Aminopeptidase I (API or, equivalently, "polypeptidase") is of special interest to the present invention. The enzyme was first isolated and characterized as a high molecular weight protein by Johnson in 1941 (Johnson, M.J., J. Biol. Chem. 137:575-586 (1941)). The $M_r$ of the native enzyme was estimated to be between 560,000 and 700,000, and the enzyme was found to consist of multiple subunits with a $M_r$ of approximately 50,000 (Johnson, M.J., J. Biol. Chem. 137:575-586 (1941); Trumbly, R.J. et al., J. Bacteriol. 156:36-48 (1983); Frey, J. et al., Biochem. Biophys. Acta 527:31-41 (1978); Metz, G. et al., Biochem. Biophys. Acta 429:933-949 (1976)).

Yeast Aminopeptidase I was established to be a glycoprotein, containing 12% carbohydrate, as well as a $Zn^{2+}$-containing metalloexopeptidase, which can be inactivated by metal-chelating agents and specifically activated by $Zn^{++}$ and $Cl^-$ (Johnson, M.J., J. Biol. Chem. 137:575-586 (1941); Trumbly, R.J. et al., J. Bacteriol. 156:36-48 (1983); Metz, G. et al., Biochem. Biophys. Acta 429:933-949 (1976); Rohm, K.H., Arch. Biochem. Biophys. 249:216-225 (1985)). However, the catalytic mechanism of API with the exception of its dependence on $Zn^{2+}$ remains unclear.

The biosynthesis of API is very sensitive to the carbon and nitrogen source in the culture medium. For example, when ammonia replaced peptone as the sole nitrogen, the activity of aminopeptidase I was enhanced 3- to 10-fold (Frey, J. et al., Biochem. Biophys. Acta 527:31-41 (1978)). Further, when glucose is used as the carbon source, the biosynthesis of API, as evidenced by in vitro translation of yeast mRNA, was decreased in early log phase, increased in late log phase, and the decreased again in stationary phase (Distel, B. et al., Biochem. Biophys. Acta 741:128-135 (1983)), although the enzymic activity increased throughout log phase and remained high in stationary phase (Frey, J. et al., Biochem. Biophys. Acta 527:31-41 (1978)).

Yeast aminopeptidase API, leucine aminopeptidase IV (Trumbly, R.J. et al., J. Bacteriol. 156:36-48 (1983)), aminopeptidase III (Matile, P. et al., Planta 96:43-53 (1971)), and aminopeptidase yscI (Rendueles, P.S. et al., FEMS Microbiol. Rev. 54:17-46 (1988); Achstetter, T. et al., Yeast 1:139-157 (1985))) have been localized to yeast vacuoles (equivalent to lysosomes found in higher eukaryotic organisms (Matile, P. et al.,

Arch. Mikrobiol. 56:148-155 (1967))) (Frey, J. et al., Biochem. Biophys. Acta 527:31-41 (1978)).

It is known that vacuolar proteases (e.g., proteinase A (Ammerer, g. et al., Mol. Cell. Biol. 6:2490-2499 (1986)), proteinase B (Moehle, C.M. et al., Mol. Cell. Biol. 7:4390-4399 (1987)), and carboxypeptidase Y (CPY) (Hasilik, A. et al., Eur. J. Biochem. 85:599-608 (1978))) are synthesized as preproproteins, and in the case of CYP, its prosequence is proven to contain the vacuolar sorting determinant (Johnson, L.M. et al., Cell 48:875-885 (1987); Valls, L.A. et al., Cell 48:87-897 (1987)). Although API had previously been localized to yeast vacuoles (Frey, J. et al., Biochem. Biophys. Acta 527:31-41 (1978)), its maturation process and intracellular sorting remained unknown.

Mutants of Saccharomyces cerevisiae incapable of effectively hydrolyzing leucine β-naphthylamide, a chromogenic substrate for aminopeptidase activity, have previously been isolated. The properties of these mutants have been attributed to a lack of one or more of four leucine aminopeptidases (so-called, LAPI-LAPIV) by Trumbly and Bradley (Trumbly, R.J. et al., J. Bacteriol. 156:36-48 (1983)). Mutations in the lap4 gene eliminated the activity of API, and the lap4 gene has been genetically mapped to the left arm of chromosome XI (Trumbly, R.J. et al., J. Bacteriol. 156:36-48 (1983)). However, since yeast strains with mutations in lap4 displayed apparently normal growth rates, API was presumed not to have a vital role in cell physiology. Alternatively, it was presumed that its absence could be compensated by other aminopeptidases (Trumbly, R.J. et al., J. Bacteriol. 156:36-48 (1983)). The lap4 gene has not been cloned and sequenced.

Aminopeptidases, in general, are important in the processing and degradation of peptides and proteins. They have been used as an alternative to Edman degradation in the determination of the amino acid sequence of polypeptides and proteins.

Substrate-specific aminopeptidases can also be used to specifically cleave amino acid residues from the amino-terminus of a protein or peptide. Thus, such enzymes may have value in removing signal sequences, nuclear localization sequences, leader sequences, secretion sequences, etc., which may be present at the amino-terminal end of a peptide or protein. Since may proteins, produced through recombinant DNA technology, or by other means contain such sequences, aminopeptidases can be used to convert such molecules into their mature and/or functional forms. The identification and availability of additional substrate-specific aminopeptidases is, therefore, highly desirable.

One aspect of the present invention concerns the cloning, sequencing, and chromosomal location of the gene encoding the API precursor protein, which contains a presequence differing from other signal sequences which direct proteins across bacterial plasma membranes and endoplasmic reticulum or into mitochondria. This cloned gene will facilitate studies of the processing, catalytic mechanism, and regulation of the biosynthesis of API.

## Brief Description of the Figures

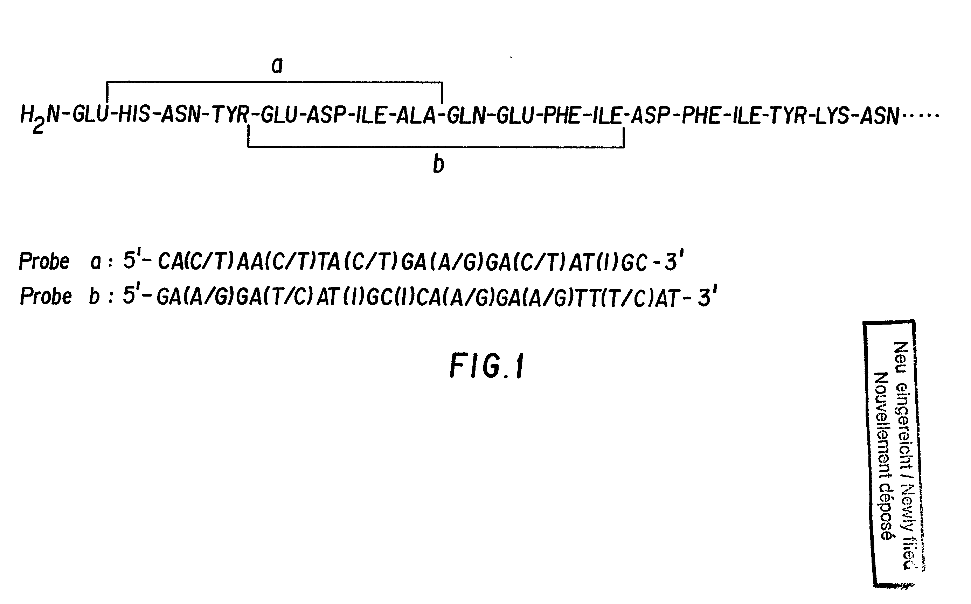

Figure 1 shows the NH₂-terminal sequence of yeast aminopeptidase I and design of synthetic oligonucleotide probes. Degenerate oligonucleotide probes a and b were designed according to the corresponding protein sequence, and were used sequentially to screen a λgt11 yeast genomic library.

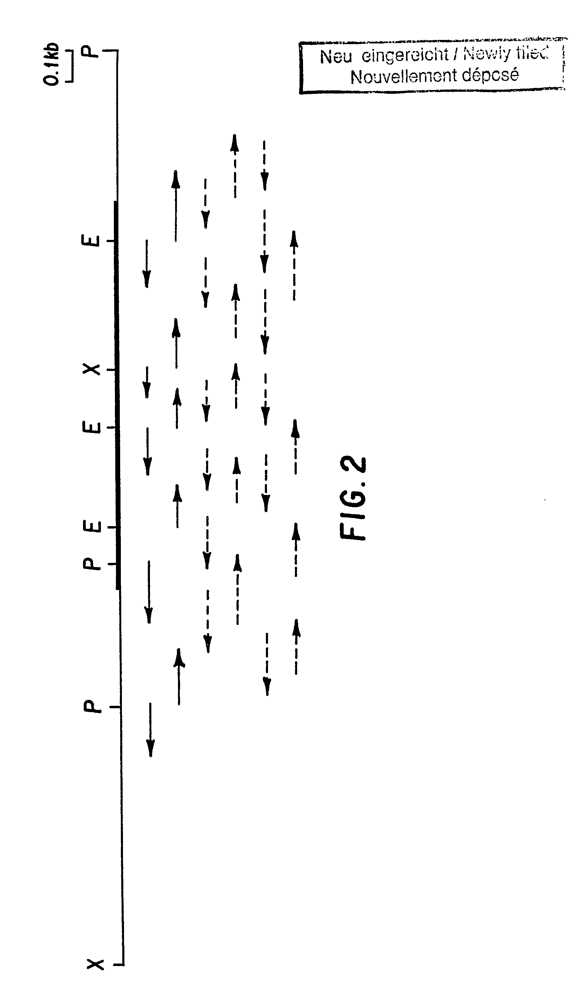

Figure 2 shows the restriction map and sequencing strategy for the genomic DNA encoding yeast aminopeptidase I. Solid arrows denote the direction and extent of T3- or T7-primer initiated DNA sequencing, and dashed arrows denote synthetic oligonucleotide-primed DNA sequencing of various subclones contained in Bluescript M13+ vector (Stratagene). The open reading frame encoding the aminopeptidase I precursor, containing 514 amino acid residues, it represented by the thick line, and the flanking regions by the thin line. Abbreviations for restriction cleavage sites are as follows: E, EcoRI; P, PstI; X, XbaI.

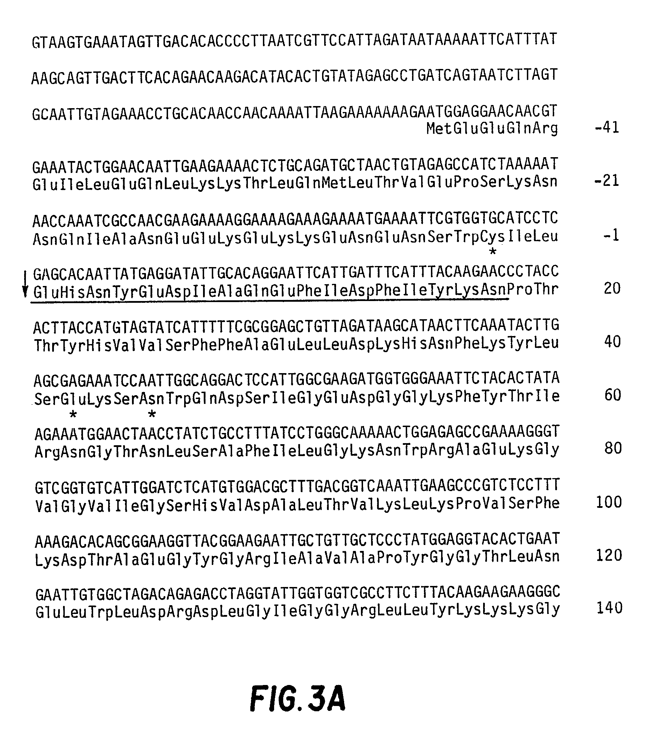

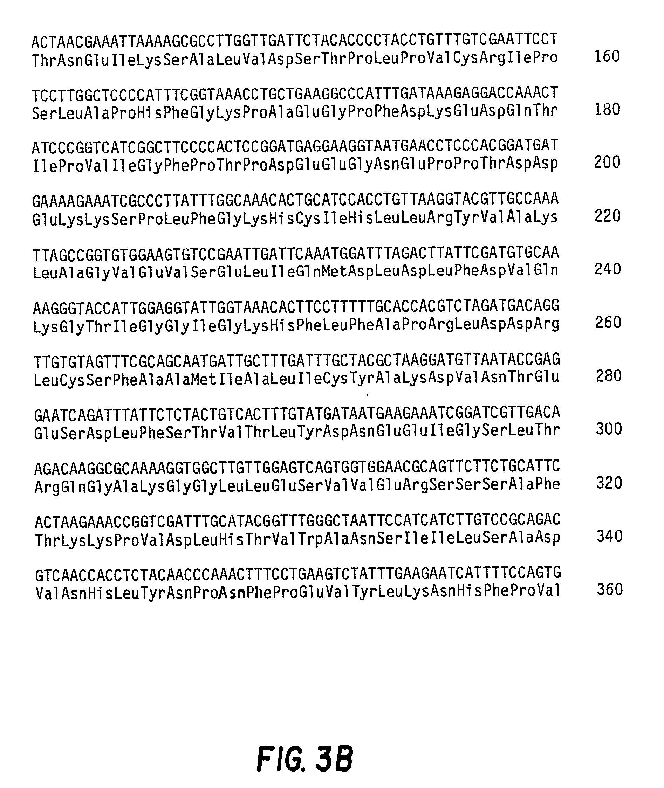

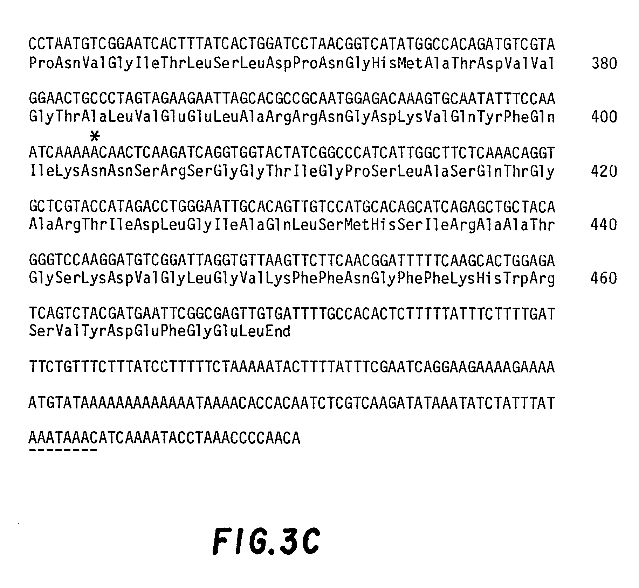

Figure 3 shows the nucleotide sequence of the genomic DNA which encodes the yeast aminopeptidase I and the deduced amino acid sequence of yeast aminopeptidase I. The clone begins with a non-coding region containing 5 in-frame stop codons. The most 5′ ATG of the open reading frame which obeyed Kozak's rule was assigned as the initiation codon. The vertical arrow marks the NH₂-terminus of the SDS-PAGE purified aminopeptidase I, and the underlined sequence was identified by protein sequence analysis (Figure 1). The open reading frame after the vertical arrow encodes a protein containing 469 amino acid residues and is terminated by a stop codon (End). The dashed underline identifies the putative polyadenylation signal, AAATAAA Asterisks identify four putative N-glycosylation sites (Asn-X-Thr or Asn-X-Ser).

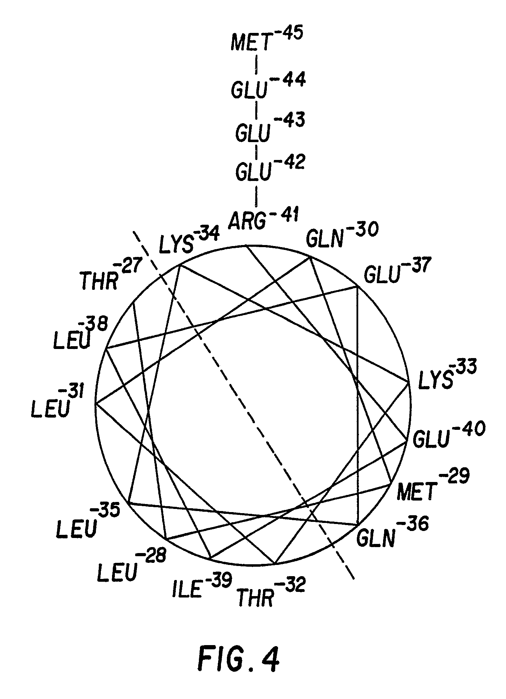

Figure 4 shows the presence of an amphiphilic α-helical structure within the presequence of yeast

3

aminopeptidase I. The first amino acid residue of the putative signal peptide of the aminopeptidase I precursor is numbered -45. The helical wheel displays residues from -41 to -27. The dotted line demarcates the hydrophobic and hydrophilic faces of the predicted amphiphilic α-helix.

## Summary of the Invention

According to this invention, substrate-specific aminopeptidases, capable of cleaving the peptide bond between the carboxyl group of a particular amino-terminal amino acid residue and the amino group of an adjacent amino acid residue were isolated and substantially purified. The invention therefore relates to the purification of these enzymes, to the uses for the enzymes, and to the substantially purified enzymes themselves.

In detail, the invention concerns a substantially purified substrate-specific aminopeptidase enzyme having an ability to cleave the peptide bond between an amino-terminal methionine residue, and every penultimate amino-terminal amino acid residue selected from the group consisting of: Arg, Asp, Gln, Gly, His, Ile, Leu, Lys, Met and Phe.

The invention further concerns a substantially purified substate-specific aminopeptidase enzyme having an ability to cleave the peptide bond between an amino-terminal residue X, and an adjacent Ile residue of a peptide, wherein X is an amino acid residue selected from the group consisting of: Ala, Arg, Gln, His, Ile, Leu, Met, Phe, Pro, Ser, Thr and Trp.

The invention further concerns a substantially purified substrate-specific aminopeptidase enzyme having an ability to cleave the peptide bond between an amino-terminal methionine residue, and every penultimate amino-terminal acid residue selected from the group consisting of: Ala, Arg, Asp, Cys, Gly, His, Ile, Leu, Lys, Met, Phe, Ser and Trp.

The invention further concerns a substantially purified substrate-specific aminopeptidase enzyme having an ability to cleave the peptide bond between an amino-terminal residue X, and an adjacent Ile residue of a peptide, wherein X is an amino acid residue selected from the group consisting of: Arg, Gln, His, Leu, Met, Phe, Pro, Ser, Thr and Trp.

The invention further concerns a substantially purified substrate-specific aminopeptidase enzyme having an ability to cleave the peptide bond between an amino-terminal methionine residue, and every penultimate amino-terminal amino acid residue selected from the group consisting of: Val, Ala, Arg, Asp, Cys, Glu, Gln, Gly, His, Ile, Leu, Lys, Met, Phe, Pro, Ser, Thr and Trp.

The invention further concerns a substantially purified substrate-specific aminopeptidase enzyme having an ability to cleave the peptide bond between an amino-terminal residue X, and an adjacent Ile residue of a peptide, wherein X is an amino acid residue selected from the group consisting of: Val, Ala, Arg, Gln, Gly, His, Ile, Leu, Met, Phe, Thr and Trp.

The invention also pertains to any of the above-described aminopeptidases which is AP2, API, or APX.

The invention also concerns a recombinant DNA molecule comprising a genetic sequence encoding any of the above-described substrate-specific aminopeptidases.

The invention also concerns any of the above-described aminopeptidases which is produced by the expression of a recombinant molecule.

The invention also concerns a presequence capable of directing a protein or polypeptide through a membrane of a yeast cell, and more specifically through such a membrane into a yeast vacuole.

The invention also concerns a recombinant nucleic acid molecule which is capable of encoding such a presequence.

The invention also provides a method for recovering any of the above-described substantially purified substrate-specific aminopeptidases from a sample containing the aminopeptidases comprising the following steps:

(a) recovering crude substrate-specific aminopeptidase from a sample containing the aminopeptidase;

(b) subjecting the crude substrate-specific aminopeptidase from step (a) to ion exchange chromatography; and

(c) recovering any substrate-specific aminopeptidase purified by the ion exchange chromatography.

The invention also pertains to the above-described method which additionally comprises the steps of:

(d) subjecting the recovered substrate-specific aminopeptidase of step (c) to hydroxylapatite chromatography; and

(e) recovering any substrate-specific aminopeptidase purified by the hydroxylapatite chromatography.

The invention also pertains to the above-described method for purifying API which additionally comprises the steps of:

(f) subjecting the recovered substrate-specific aminopeptidase of step (e) to Phenyl-Sepharose CL-4B chromatography; and

(g) recovering any substrate-specific aminopeptidase purified by the Phenyl-Sepharose CL-4B chromatography.

The invention also provides a method for removing an amino-terminal methionine residue from a peptide containing the amino-terminal residue, and containing as a penultimate amino-terminal amino acid residue, an amino acid selected from the group consisting of: Arg, Asp, Gln, Gly, His, Ile, Leu, Lys, Met, and Phe, which method comprises incubating the peptide in the presence of the substrate-specific aminopeptidase AP2 to thereby remove the amino-terminal residue.

The invention also provides a method for removing an amino-terminal methionine residue from a peptide containing the amino-terminal residue, and containing as a penultimate amino-terminal amino acid residue, an amino acid selected from the group consisting of Ala, Arg, Asp, Cys, Gly, His, Ile, Leu, Lys, Met, Phe, Ser and Trp, which method comprises incubating the peptide in the presence of the substrate-specific aminopeptidase API to thereby remove the amino-terminal residue.

The invention also provides a method for removing an amino-terminal methionine residue from a peptide containing the amino-terminal residue, and containing as a penultimate amino-terminal amino acid residue, an amino acid selected from the group consisting of Val, Ala, Arg, Asp, Cys, Glu, Gln, Gly, His, Ile, Leu, Lys, Met, Phe, Pro, Ser, Thr and Trp, which method comprises incubating the peptide in the presence of the substrate-specific aminopeptidase APX to thereby remove the amino-terminal residue.

The invention additionally provides a method for removing an amino-terminal residue X from a peptide containing the terminal residue X, and an adjacent Ile residue, wherein X is selected from the group consisting of Ala, Arg, Gln, His, Ile, Leu, Met, Phe, Pro, Ser, Thr and Trp, which method comprises incubating the peptide in the presence of the substrate-specific aminopeptidase AP2 to thereby remove the amino-terminal residue.

The invention additionally provides a method for removing an amino-terminal residue X from a peptide containing the terminal residue X, and an adjacent Ile residue, wherein X is selected from the group consisting of Arg, Gln, His, Leu, Met, Phe, Pro, Ser, Thr and Trp, which method comprises incubating the peptide in the presence of the substrate-specific aminopeptidase API to thereby remove the amino-terminal residue.

The invention additionally provides a method for removing an amino-terminal residue X from a peptide containing the terminal residue X, and an adjacent Ile residue, wherein X is selected from the group consisting of Val, Ala, Arg, Gln, Gly, His, Ile, Leu, Met, Phe, Thr and Trp, which method comprises incubating the peptide in the presence of the substrate-specific aminopeptidase APX to thereby remove the amino-terminal residue.

The invention further concerns the embodiment of any of the above methods wherein the substrate-specific aminopeptidase is immobilized to a solid support.


## Detailed Description of the Invention


I. Isolation, and Purification of the Substrate-Specific Aminopeptidases AP2, API and APX of the Present Invention

In accordance with the present invention, any of the substrate-specific aminopeptidases of the present invention can be isolated from a sample containing the enzyme. Substrate-specific aminopeptidases appear to be ubiquitous, being found in all eukaryotes, including plants, animals, and multicellular organisms. A different enzyme having methionine-specific aminopeptidase acitivty is also believed to be present in prokaryotes. Therefore, any cell source is contemplated in this invention. As used herein, the sample containing any of the enzymes will be referred to simply as "sample." Unless specified as being unique to one or the other of the substrate-specific aminopeptidases of the present invention, the recombinant DNA methods described herein can be employed to clone and express any or all of the aminopeptidases of the present invention.

In describing the present invention, certain abbreviations are employed. The abbreviations used are: API, aminopeptidase I; B buffer, a buffer containing 20 mM HEPES-K$^+$, pH 7.35, 5 mM MgCl$_2$, 0.5 mM DTT, 0.2 M KCl, and 0.02% NaN$_3$, bp, base pair; BSA, bovine serum albumin; CNBr, cyanogen bromide; CPY, carboxypeptidase Y; DEAE, diethylaminoethyl; DTT, dithiothreitol; HEPES, N-2-hydroxyethylpiperazine-N'-2-ethanesulfonic acid; kb, kilobase; PBS, 0.15 M NaCl, 12.5 mM $KH_2PO_4$, pH 7.4, 0.02% $NaN_3$; pNA, p-nitroanilide; Pth, 3-phenyl-2-thiohydantoin; SDS, sodium dodecyl sulfate; SDS-PAGE, sodium dodecyl sulfate-polyacrylamide gel electrophoresis; SSC, 0.15 M NaCl, 15 mM sodium citrate, pH 7.0; YPD, medium containing 1% yeast extract, 2% Bacto-peptone, and 2% glucose.

In describing the present invention, reference is made to a number of biological materials, vectors, and host strains. All of the materials used to practice this invention are commercially available or are widely available to those of ordinary skill. Except as indicated, alternative materials, vectors and strains can be employed in order to practice the present invention. Yeast extract and Bactopeptone were from Difco Laboratories. Protein assay reagent (Bradford method) and SDS-PAGE electrophoresis reagents were from BioRad, Inc. Methionine p-nitroanilide (Met-pNA) was from Bachem, Inc. Bovine serum albumin (BSA), HEPES, dithiothreitol (DTT), glucose, sorbitol, Tris, glycerol (enzyme grade), lyticase, herring sperm DNA, protein molecular weight markers, and Coomassie blue were purchased from Sigma Chemical Corp. DEAE Sepharose CL-6B, Sepharose CL-6B, and CNBr-activated Sepharose 4B were from Pharmacia. PM-30 membrane was from Amicon. Constant boiling (6 N) HCl and Polybrene were from Pierce Biochemicals. Reagents and solvents for amino acid analysis and Ready-solv EP cocktails were from Beckman Instruments. Reagents and solvents for protein sequence analysis and DNA synthesis were from Applied Biosystems, Inc. [$^{32}$P]-labeled nucleotides were from New England Nuclear. Restriction enzymes, E. coli DNA polymerase I (Klenow fragment), T4 polynucleotide kinase, RNase A, T4 DNA ligase and calf intestine alkaline phosphatase were from New England Biolabs or Boehringer Mannheim. [$\alpha$-$^{35}$S]dATP was from Amersham. Nitrocellulose membranes for screening and for immunoblot analysis were from Millipore and Schleicher & Schuell. X-ray film was from Kodak. Phenol and ammonium sulfate were from BRL. Saccharomyces chromo-di-hybridizer was from Clonetech. Freund's complete and incomplete adjuvant was from Miles Laboratories. PicoBlue immunodetection kit was from Stratagene. All other chemicals were reagent grade or better.

Bluescript M13+ and its host stain E. coli DH5$\alpha$ [recA$^-$, F$^-$, endtl, gyrA96, thi-1, hsdR17 ($r_k^-$, $m_k^+$), sup44, relA1] were from Stratagene. Y1088 ($\Delta$lacU169, supE, SupF, hsdR$^-$, hsdM$^-$, metB, trpR, tonA21, proC::Tn5 (pMC9)] and LE392 (F$^-$, supE44, supF58, lacY1, galT22, metB1, trpR55, hsdR514 ($R_k^-$, $M_k^-$)] were used as hosts for $\lambda$gt11 and EMBL3A, respectively. Bakers' yeast strain TD71.8 ($\alpha$, ino3, leu2, his3, lys2) was a gift from Dr. Jack Szostak. The $\lambda$gt11 and EMBL3A yeast genomic libraries were gifts from Dr. Richard A. Young. Similar vectors and libraries can be alternatively employed.

In the following description of the purification of the substrate-specific aminopeptidases of the present invention, the activity of the aminopeptidase may be assayed by any convenient method. The preferred assay for substrate-specific aminopeptidase activity is as follows:

Enzyme activity can be assayed using either Met-pNA (methionine para-nitroanilide) or the peptide, Met-Ala-Ser, as the substrate. Met-pNA was used to assay for the aminopeptidase activity of API. For this purpose, the hydrolysis of the p-nitroanilide substrate was assayed according to Tuppy et al. (Tuppy, H. et al., Physiol. Chem. 329:278-288 (1962)).

When Met-pNA is used as the substrate, an aliquot of 10 $\mu$l from each fraction collected from a chromatographic column is added to a labeled well of a microtiter plate containing 45 $\mu$l of a solution which contains 4 mM Met-pNA, 10 mM HEPES-K$^+$, pH 7.35, 0.2 mM $CoCl_2$, 0.2 M KCl, 1.5 mM $MgCl_2$, 0.02% $NaN_3$. All of the reactions are preferably conducted using a microtiter plate. After incubation for one hour at room temperature, the absorbance of the sample at 414 nm is determined using a plate reader.

When the assay is conducted using Met-Ala-Ser, as the substrate an aliquot of 10 $\mu$l from each fraction collected from a chromatographic column is transferred to a labeled well of a microtiter plate containing 45 $\mu$l of the solution which contains 4 mM Met-Ala-Ser, 10 mM HEPES-K$^+$, pH 7.35, 0.2 mM $CoCl_2$, 0.2 KCl, 1.5 mM $MgCl_2$, 0.02% $NaN_3$, 10 $\mu$g of L-amino acid oxidase, 115 $\mu$g of horseradish peroxidase and 10 $\mu$g of a o-dianisidine dihydrochloride. The reactions are carried out at room temperature, for one hour, and the absorbance at 450 nm, or equivalently at 414 nm, is recorded.

UV measurements, when required, were obtained using a Hewlett-Packard 8450A UV spectrophotometer. Protein assays were performed by the method of Bradford(Valls, L.A. et al., Cell 48:87-897 (1987)), using BSA as the protein standard. Radioactive samples were counted on a Beckman LS 3801 scintillation counter.

The isolation and purification of the substrate-specific aminopeptidases of the present invention will hereinafter be described from a yeast sample, although it is to be understood that other samples could be used as the source material.

Yeast cells, preferably Saccharomyces cerevisiae, and most preferably strain TD71.8, are grown in suitable medium (preferably YPD medium: 1% yeast extract, 2% Bacto-peptone, 2% glucose) to an $A_{600}$ of 5-20. Preparations of cells harvested at an $A_{600}$ of 8, or at an $A_{600}$ of 18-29 are found to have equivalent substrate-specific aminopeptidase activity. Cells are harvested, preferably by centrifugation, and resuspended in YPD medium containing 15% glycerol. The cells may then be frozen to -70 °C, and stored for an extended period.

Frozen cells are thawed, preferably by incubation in a 30 °C water bath, and collected by centrifugation at room temperature. The pelleted cells are resuspended in a suitable buffer such as Buffer S (1 M Sorbitol, 50 mM Tris, pH 7.8, 10 mM $MgCl_2$, 3 mM DTT), supplemented with lyticase, and incubated at 30 °C, to permit spheroplast formation. The spheroplasts are then collected, washed in additional Buffer S, lysed and homogenized in hypotonic buffer, such as buffer A (10 mM HEPES-K$^+$, pH 7.0, 1.5 pM $MgCl_2$, 10 mM KCl, and 0.5 mM DTT). Homogenization is preferably accomplished at 4 °C using a Dounce homogenizer. Yeast substrate-specific aminopeptidase is released into the cellular lysate by gentle shaking. After removing any debris by centrifugation, glycerol is added to the supernatant of the lysate (10% final concentration) in order to increase the stability of the substrate-specific aminopeptidase.

The above-described supernatant of the cellular lysate is then concentrated to 100 ml by the use of a PM-10 ultrafiltration membrane and dialyzed three times against 2 liters of a suitable buffer, such as Buffer H (10 mM HEPES-K$^+$, pH 7.35, 1.5 mM $MgCl_2$, 0.5 mM DTT, 0.2% $NaN_3$, 10% glycerol), supplemented to contain 0.05 M KCl. Residual cell biomaterials in the supernatant are removed to avoid protein-biomaterial aggregation in subsequent steps. Ion exchange chromatography can be used for this procedure. In this step, the ion exchange used is preferably CM-Sepharose chromatography, most preferably employing CM-Sepharose CL-6B. The concentrated supernatant is preferably loaded on a CM-Sepharose CL-6B column which has been connected to a DEAE-Sepharose CL-6B column such that the eluate from the CM-Sepharose CL-6B column is applied to the DEAE-Sepharose CL-6B column continually. Both columns are equilibrated against the same buffer as that used for dialysis. The two connected columns are then eluted with 2 liters of buffer H (supplemented to contain 0.05 KC1), or another suitable buffer. The columns are then disconnected.

The DEAE-Sepharose CL-6B column is eluted with a linear gradient of 0.05 M to 0.5 M KCl in buffer H at approximately 17 ml/hr. Fractions of approximately 5 ml are collected and assayed for substrate-specific aminopeptidase activity. The fractions which contain aminopeptidase activity (typically fractions 41-71 of 143 total fractions, average conductivity 9.7 ms) are pooled and concentrated to a volume of 10 ml using a PM-10 ultrafiltration membrane.

The concentrated eluate from the above-described DEAE-Sepharose CL-6B chromatography is dialyzed three times against 1 liter of buffer H (supplemented to contain 0.05 M KCl) and applied to a second DEAE-Sepharose CL-6B column equilibrated with the same buffer as that used for dialysis. This column is eluted with a linear gradient of 0.05 M to 0.2 M KCl in buffer H at 18 ml/hr. Fractions of 4-5 ml are collected and assayed for substrate-specific aminopeptidase activity. Aminopeptidase AP2, API and APX activity are all found in fractions 113-133 (of 143 total fractions); the conductivity of the pooled fractions is approximately 10.2 ms. These fractions are pooled and concentrated to 5 ml by a PM-10 ultrafiltration membrane.

The concentrated eluate from the above-described DEAE-Sepharose CL-6B chromatography is dialyzed three times against 1 liter of potassium phosphate buffer, pH 7.15, 0.5 M DTT, 5 mM KCl, 10% glycerol, 0.02% $NaN_3$, and applied to a hydroxylapatite column equilibrated against the same buffer as that used for dialysis. The column is eluted with a linear gradient of 0.01 M to 0.5 M potassium phosphate buffer, pH 7.15, 0.5 M DTT, 5 mM KCl, 10% glycerol, 0.02% $NaN_3$, at 18 ml/hr. Fractions of 4-5 ml are collected. Aminopeptidase AP2 is found in fractions 43-47 (average conductivity 7.2 ms). Aminopeptidase APX is found in fractions 67-75 (average conductivity 16.5 ms). Aminopeptidase API is found in fractions 83-97 (average conductivity 23.0 ms). Fractions containing each of the aminopeptidases are separately pooled, and concentrated to a volume of 5 ml using PM-10 ultrafiltration membrane.

Aminopeptidase API may be further purified using hydrophobic interaction chromatography (preferably using Phenyl-Sepharose CL-4B chromatography). This is preferably accomplished by dialyzing the concentrated eluate from the above-described hydroxylapatite chromatography three times against 1 liter of buffer H (10 mM HEPES-K$^+$, pH 7.35, 0.5 mM DTT, 1.5 mM $MgCl_2$, 0.02% $NaN_3$, 10% glycerol) containing 0.2 M KCl. An equal volume of 80% saturated ammonium sulfate solution, 10 mM HEPES-K$^+$, pH 7.35, 10% glycerol, 0.02% $NaN_3$ are added to the solution containing aminopeptidase. This mixture is applied to a Phenyl-Sepharose CL-4B column equilibrated with 40% saturated ammonium sulfate solution, 10 mM HEPES-K$^+$, pH 7.35, 10% glycerol, 0.25 mM DTT, 0.1 M KCl, 0.02% $NaN_3$. The column is first eluted with 50 ml of the same buffer described above and then with a linear gradient of 40% to 0% ammonium sulfate solution, 10 mM HEPES-K$^+$, pH 7.35, 10% glycerol, 0.25 mM DTT, 0.02% $NaN_3$, 100 mM KCl at approximately 72 ml/hr. Fractrions of approximately 4 ml are collected and assayed for aminopeptidase activity. Aminopeptidase API is typically found in fractions 103-113 of 141 fractions; the active fractions are pooled (average conductivity approximately 26 ms), and concentrated to 3 ml using a PM-10 ultrafiltration membrane.

Using this series of chromatography steps, yeast substrate-specific aminopeptidase AP2 and API were purified to near homogeneity. The enzymes have apparent molecular weights of approximately 93,000 and 50.000, respectively.

In an alternative method for purifying API, a crude yeast extract fom concentrated cells can be obtained according to the method of Lin et al. (Lin, R.J. et al., J. Biol. Chem. 260:14780-14792 (1985)). The API present in such a sample can be purified to near homogeneity by the method of Metz and Rohm (Metz, T. et al., Biochem. Biophys. Acta 429:933-949 (1976)). Aliquots of the concentrated eluate from the Sepharose CL-6B chromatography are preferably applied to three SDS- PAGE gels (8%) with a thickness of 1.5 mm and with a well size of 12 cm, electrophoresed, and electroeluted, as described by Hunkapiller, M.W. et al. - (Methods Enzymol. 91:227-247 (1983)). When this alternative purification method was employed, the 2 liters of concentrated cells were found to contain approximately 900 µg of API. A purification of approximately 800-fold was achieved by this method and a yield of -350 µg of API was realized.

The terms peptide, polypeptide, and protein are all intended to refer to polymers of amino acid residues. A peptide is a polymer of at least two amino acid residues joined to one another by a peptide bond. A polypeptide is a polymer of several amino acid residues joined to one another by a peptide bond. A protein is a large polymer of many amino acid residues joined to one another by a peptide bond.

The term "aminopeptidase" as used herein is intended to refer to an enzyme capable of attacking and cleaving the peptide bond which joins the amino-terminal amino acid residue of a peptide (and thus also of a polypeptide or protein) to the penultimate amino-terminal amino acid residue of that peptide (or polypeptide or protein).

The aminopeptidases of the present invention are "substrate-specific aminopeptidases." An aminopeptidase is said to be "substrate-specific" if only certain peptides or proteins (i.e. not every peptide or protein) can serve as a substrate for the enzyme. Such suitable substrates possess particular amino-terminal and penultimate amino-terminal residues, which are joined by a peptide bond. The substrate specificity of the aminopeptidase is defined by the ability or inability to cleave a particular substrate. The substrate specificity of the aminopeptidases of the present invention is defined below.

The term "presequence" is intended to refer to a polypeptide which, when linked via a peptide bond to the amino terminus of a protein (or a second polypeptide), is sufficient to cause the linked protein or polypeptide to be directed to a particular cellular location. Of special concern to the present invention are presequences which are sufficient to direct a protein or polypeptide through a membrane of a yeast cell, and more specifically through such a membrane into a yeast vacuole. The preferred sequence of such a "vacuolar localization presequence" is:

Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu.

A vacuolar localization sequence may, however, contain or lack one, two or more additional amino acids from either of its termini. For example, the sequence may contain an additional methionine residue at its amino terminus. A vacuolar localization presequence may additionally be capable of directing a linked protein or polypeptide to organelles other than vacuoles, or through membranes other than the vacuolar membrane.

The elucidation of the sequence of the localization presequence permits one to link the presequence to any desired heterologous protein or polypeptide in order to cause the protein or polypeptide to be subject to the control of the localization presequence. As used herein, a protein or polypeptide is said to be "heterologous" if it is not naturally found to be linked to the vacuolar localization presequence of the present invention. Heterologous proteins and polypeptides which are linked to the vacuolar localization presequence of the present invention can be prepared through the use of recombinant techniques such as by linking a gene sequence capable of encoding the localization presequence to a gene sequence capable of encoding the heterologous protein or polypeptide. Procedures for constructing recombinant molecules in accordance with the above-described method are disclosed by Maniatis, T., et al., In: Molecular Cloning, A Laboratory Manual, Cold Spring Harbor Press, Cold Spring Harbor, NY (1984), which reference is herein incorporated by reference.

The sequence of amino acid residues in a protein or polypeptide is designated herein either through the use of their commonly employed 3-letter designations. A listing of these 3-letter designations may be found in textbooks such as Biochemistry, Lehninger, A., Worth Publishers, New York, NY (1970). When such a sequence is listed horizontally, the amino-terminus is intended to be on the left end whereas the carboxyl-terminus is intended to be at the right end. The residues of amino acids in a peptide may be indicated by capitalizing the first letter of the amino acid residue, or alternatively, by separating adjacent residues using hyphens. Such hyphens are intended solely to facilitate the presentation of a sequence. As a purely illustrative example, the amino acid sequence designated:

Gly-Ala-Ser-Phe

indicates that an Ala residue is linked to the carboxyl group of Gly, and that a Ser residue is linked to the carboxyl group of the Ala residue and to the amino group of a Phe residue. The designation further indicates that the amino acid sequence contains the tetrapeptide Gly-Ala-Ser-Phe. The designation is not intended to limit the amino acid sequence to this one tetrapeptide, but is intended to include (1) the tetrapeptide having one or more amino acid residues linked to either its amino or carboxyl end, (2) the tetrapeptide having one or more amino acid residues linked to both its amino and its carboxyl ends, (3) the tetrapeptide having no additional amino acid residues.

The proteins and peptides of the present invention may be encoded by nucleic acid molecules. The sequence of such nucleic acid molecules can readily be determined by those of ordinary skill by reference to the genetic code (Watson, J.S., In: Molecular Biology of the Gene, 3rd Ed., W.A. Benjamin, Inc., Menlo Park, CA (1977), pp. 356-357); Lehninger, A., Biochemistry, Worth Publishers, New York, NY (1970)). Because the genetic code is degenerate, more than one codon may be used to encode a particular amino acid.

The present invention concerns a substrate-specific aminopeptidase enzyme which is "substantially pure" or which has been "substantially purified". As used herein, the terms "substantially pure" or "substantially purified" are intended to be equivalent, and to describe a substrate-specific aminopeptidase which is substantially free of a compound normally associated with the enzyme in its natural state, i.e., a protein, carbohydrate, lipid, etc. The term is further meant to describe a substrate-specific aminopeptidase which is homogeneous by one or more of the assays of purity or homogeneity used by those of skill in the art. For example, a substantially pure substrate-specific aminopeptidase will show constant and reproducible characteristics within standard experimental deviations for parameters such as the following: molecular weight, chromatographic techniques, etc. The term "substantially pure," however, is not meant to exclude artificial or synthetic mixtures of the enzymes with other compounds. The term is also not meant to exclude the presence of impurities which do not interfere with the biological activity of the enzyme, and which may be present, for example, due to incomplete purification.

## II. Characterization of the Substrate-Specific Aminopeptidases of the Present Invention

### The Aminopeptidase AP2

SDS-polyacrylamide gels of the purified sample reveal that AP2 migrates as a single band of about apparent molecular weight 93,000 when stained with Coomassie blue. The electrophoresis was performed according to the method of Laemmli, U.K. (Nature 227:680-685 (1970)) using either a 9 or 10% gel. The gel was stained with Coomassie blue. The gel was calibrated using the following molecular weight standards: myosin (205,000), E. coli $\beta$-galactosidase (116,000), rabbit muscle phosphorylase (97,000), bovine serum albumin (66,000), egg albumin (45,000), and carbonic anhydrase (29,000).

### The Aminopeptidase API

Yeast Aminopeptidase API is the preferred aminopeptidase of the present invention. As used herein, the equivalent terms "Aminopeptidase API" or "Yeast aminopeptidase I" are intended to refer to the yeast aminopeptidase which was referred to as "Yeast aminopeptidase 121" or "AP121" in Applicants' U.S. Patent Application Serial No. 07/243,733, filed on September 13, 1988, from which application the present application claims priority. The name of the aminopeptidase has been changed in order to conform to the nomenclature employed by those of ordinary skill in this field.

SDS-polyacrylamide gels of the API sample, purified as described above, reveal that API migrates as a single band of about apparent molecular weight 50,000 ± 2,000 when stained with Coomassie blue. The electrophoresis was performed as described above.

The native molecular weight of the enzyme was determined as follows: Partially purified enzyme was applied to a Sepharose CL-6B (2.0 x 90 cm). The elution buffer was B buffer (20 mM HEPES-K$^+$, pH 7.35, 5 mM MgCl$_2$, 0.5 mM DTT, 0.2 M KCl, 0.02% NaN$_3$), and the flow rate was 14 ml/h. The elution volume of aminopeptidase I was determined by the standard enzyme assay, and the apparent molecular weight was calculated from the relative elution volumes of protein standards including (1) thyroglobulin (669,000), (2) apoferritin (443,000), (3) β-amylase (200,000), (4) alcohol dehydrogenase (150,000), (5) bovine serum albumin (66,000), and (6) carbonic anhydrase (29,000). The $M_r$ of the native enzyme was found to be about 570,000 ± 20,000.

The $M_r$ of the native enzyme (570,000 ± 20,000) and its subunit (50,000 ± 2,000) agree with the results of others, who determined that the protein was a multimeric glycoprotein (Johnson, M.J., J. Biol. Chem. 137:575-586 (1941); Trumbly, R.J. et al., J. Bacteriol. 156:36-48 (1983); Frey, J. et al., Biochem. Biophys. Acta 527:31-41 (1978); Metz, G. et al., Biochem. Biophys. Acta 429:933-949 (1976)). In order to clone the encoding genomic DNA, the $NH_2$-terminal sequence of the first 18 amino acids of the enzyme was determined, and this sequence formed the basis for the synthesis of oligonucleotide probes (Figure 1).

To determine the amino acid sequence of the $NH_2$-terminus of the protein, purified API aminopeptidase was subjected to Edman degradation in order to determine the amino acid sequence of the amino-terminus of the enzyme. This analysis revealed that the amino terminus was composed of the following sequence: GLU-HIS-ASN-TYR-GLU-ASP-ILE-ALA-GLN-GLU-PHE-ILE-PHE-ILE-TYR-LYS-ASN.

III. Substrate-Specificity of the Aminopeptidases

A partial determination of substrate-specificity of AP2, API and APX was carried out using a set of thirty six pure peptides. The peptides had either of two sequences:

(I)     MET-X-ILE-PRO-GLU-THR

or

(II)     X-ILE-PRO-GLU-THR

wherein "X" refers to one of the following eighteen amino acids: Val, Ala, Arg, Asp, Cys, Glu, Gln, Gly, His, Ile, Leu, Lys, Met, Phe, Pro, Ser, Thr and Trp.

AP2 was found to be able to cleave those peptides of group I wherein "X" was either Arg, Asp, Gln, Gly, His, Ile, Leu, Lys, Met or Phe. No other tested peptide of group I could be cleaved by AP2. AP2 was able to cleave only those peptides of group II wherein "X" was Ala, Arg, Gln, His, Ile, Leu, Met, Phe, Pro, Ser, Thr or Trp. No other tested peptide of group II could be cleaved.

API was found to be able to cleave those peptides of group I wherein "X" was either Ala, Arg, Asp, Cys, Gly, His, Ile, Leu, Lys, Met, Phe, Ser or Trp. No other tested peptide of group I could be cleaved by API. API was able to cleave only those peptides of group II wherein "X" was Arg, Gln, His, Leu, Met, Phe, Pro, Ser, Thr or Trp. No other tested peptide of group II could be cleaved.

APX was found to be able to cleave any of the tested peptides of group I. APX was able to cleave only those peptides of group II wherein "X" was Val, Ala, Arg, Gln, Gly, His, Ile, Leu, Met, Phe, Thr or Trp. No other tested peptide of group II could be cleaved.

IV. Genetic Engineering of the Aminopeptidases of the Present Invention

The identification of fractions which contain substantially purified AP2, API or APX activity permits the amino acid sequence of these enzymes to be determined, and further permits these molecules to be produced through the application of recombinant DNA techniques. Thus, the present invention includes not only substantially purified AP2, API and APX, and methods for their use, but also includes the amino acid sequences of these enzymes, the genetic sequences encoding these enzymes, vehicles containing such genetic sequences, hosts transformed therewith, enzyme production by transformed host expression, and utilization of the enzymes in the cleavage of substrate-containing peptides or proteins. API is the preferred substrate-specific aminopeptidase of the present invention.

In order to obtain the amino acid sequence of the enzymes, the enzymes in the substantially purified fractions are recovered as by electroelution from a polyacrylamide gel. The recovered molecules may then be sequenced, preferably using an automated sequenator, and the amino acid sequence of the enzyme thereby determined.

Alternatively, and more preferably, the amino acid sequence of the enzymes is determined through an analysis of the DNA or, more preferably, the cDNA sequence, of the gene which encodes the enzyme. In order to obtain these nucleic acid sequences, a source which contains the AP2, API, or APX gene or cDNA sequence is screened with oligonucleotide probes which encode fragments of the AP2, API or APX enzyme.

In order to prepare the oligonucleotide probes, the substantially purified enzyme is recovered and fragmented as with cyanogen bromide, or with proteases such as papain, chymotrypsin, trypsin, etc. (Oike, Y., et al., J. Biol. Chem. 257:9751-9758 (1982); Liu, C., et al., Int. J. Pept. Protein Res. 21:209-215 (1983)).

The resulting peptides are separated, preferably by reverse-phase HPLC, and subjected to amino acid sequencing. To accomplish this task, the peptides are preferably analyzed by automated sequenators.

Once one or more suitable peptide fragments have been sequenced, the DNA sequences capable of encoding them are examined. If the peptides are greater than 10 amino acids long, this sequence information is generally sufficient to permit one to clone a gene sequence such as those which encode the enzymes of the present invention. Because the genetic code is degenerate, however, more than one codon may be used to encode a particular amino acid (Watson, J.D., In: Molecular Biology of the Gene, 3rd Ed., W.A. Benjamin, Inc., Menlo Park, CA (1977), pp. 356-357). Using the genetic code, one or more different oligonucleotides can be identified, each of which would be capable of encoding the enzyme peptides. The probability that a particular oligonucleotide will, in fact, constitute the actual enzyme fragment-encoding sequence can be estimated by considering abnormal base pairing relationships and the frequency with which a particular codon is actually used (to encode a particular amino acid) in eukaryotic cells. Such "codon usage rules" are disclosed by Lathe, R., et al., J. Molec. Biol. 183:1-12 (1985). Using the "codon usage rules" of Lathe, a single oligonucleotide, or a set of oligonucleotides, that contains a theoretical "most probable" nucleotide sequence capable of encoding the enzyme fragment's peptide sequences is identified. Techniques for synthesizing oligonucleotides are disclosed by, for example, Wu, R., et al., Prog. Nucl. Acid. Res. Molec. Biol. 21:101-141 (1978)). Procedures for constructing recombinant molecules in accordance with the above-described method are disclosed by Maniatis, T., et al., In: Molecular Cloning, A Laboratory Manual, Cold Spring Harbor Press, Cold Spring Harbor, NY (1984), which reference has been herein incorporated by reference.

Although occasionally an amino acid sequence may be encoded by only a single oligonucleotide, frequently the amino acid sequence may be encoded by any of a set of similar oligonucleotides. Importantly, whereas all of the members of this set contain oligonucleotides which are capable of encoding the peptide fragment and, thus, potentially contain the same oligonucleotide sequence as the gene which encodes the peptide fragment, only one member of the set contains the nucleotide sequence that is identical to the nucleotide sequence of the gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

The oligonucleotide, or set of oligonucleotides, containing the theoretical "most probable" sequence capable of encoding the enzyme fragment peptide is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate a gene sequence which encodes the enzyme (Maniatis, T., et al., Molecular Cloning, A Laboratory Manual, Cold Spring Harbor Press, Cold Spring Harbor, NY (1982)).

The DNA probe may be labeled with a detectable group. Such detectable group can be any material having a detectable physical or chemical property. Such materials have been well-developed in the field of immunoassays and in general most any label useful in such methods can be applied to the present invention. Particularly useful are enzymatically active groups, such as enzymes (see Clin. Chem. 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescers (see Clin. Chem. 25:353 (1979)); chromophores; luminescers such as chemiluminescers and bioluminescers (see Clin. Chem. 25:512 (1979)); specifically bindable ligands; proximal interacting pairs; and radioisotopes such as $^3$H, $^{35}$S, $^{32}$P, $^{125}$I and $^{14}$C. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to, beta-galactosidase, alkaline phosphatase and peroxidase.

A suitable oligonucleotide, or set of oligonucleotides, which is capable of encoding a fragment of the gene sequence which encodes the enzyme (or which is complementary to such an oligonucleotide, or set of oligonucleotides) is identified (using the above-described procedure), synthesized, and hybridized by means well known in the art, against a DNA or, more preferably, a cDNA preparation derived from cells which are capable of expressing the enzyme. Single-stranded oligonucleotide molecules complementary to the "most probable" enzyme peptide encoding sequences can be synthesized using procedures which are well known to those of ordinary skill in the art (Belagaje, R., et al., J. Biol. Chem. 254:5765-5780 (1979); Maniatis, T., et al., In: Molecular Mechanisms in the Control of Gene Expression, Nierlich, D.P., et al., Eds., Acad. Press, NY (1976); Wu, R., et al., Prog. Nucl. Acid Res. Molec. Biol. 21:101-141 (1978); Khorana, R.G.,

Science 203:614-625 (1979)). Additionally, DNA synthesis may be achieved through the use of automated synthesizers. Techniques of nucleic acid hybridization are disclosed by Maniatis, T., et al. (In: Molecular Cloning, A Laboratory Manual, Cold Spring Harbor Laboratories, Cold Spring Harbor, NY (1982)), and by Haymes, B.D., et al. (In: Nucleic Acid Hybridization, A Practical Approach, IRL Press, Washington, DC (1985)), which references are herein incorporated by reference.

A DNA sequence which encodes AP2, API or APX may be derived from a variety of sources. For example, mRNA encoding any of these enzymes may be isolated from the tissues of any species that produces the enzyme, and identified using the Northern blot method (Alwine et al., Method Enzymol. 68:220-242 (1979)) and labeled oligonucleotide probes. The mRNA may then be converted to cDNA by techniques known to those skilled in the art. Alternatively, genomic DNA may be isolated and employed. The source of DNA or cDNA used will preferably have been enriched for the gene sequence which encodes the enzyme. Such enrichment can most easily be obtained from cDNA obtained by extracting RNA from cells, such as yeast cells, which produce high levels of the enzyme. An example of such a cell is a Saccharomyces cerevisiae cell.

Any of a variety of methods may be used to clone a gene sequence which encodes the enzymes of the present invention. One such method entails analyzing a shuttle vector library of cDNA inserts (derived from a cell that expresses the enzymes of the present invention) for the presence of an insert which contains a gene sequence which is capable of encoding the enzyme. Such an analysis may be conducted by transfecting cells with the vector, and then assaying for enzyme expression.

To identify and clone the gene sequence capable of encoding any of the enzymes of the present invention, a DNA, or more preferably a cDNA, library is screened for its ability to hybridize with the oligonucleotide probes described above. Suitable DNA preparations (such as human genomic DNA) are enzymatically cleaved, or randomly sheared, and ligated into recombinant vectors. The ability of these recombinant vectors to hybridize to the above-described olignucleotide probes is then measured. Vectors found capable of such hybridization are then analyzed to determine the extent and nature of the enzyme sequences which they contain. Based purely on statistical considerations, a gene sequence capable of encoding any of the enzymes of the present invention could be unambiguously identified (via hybridization screening) using an oligonucleotide probe having only 18 nucleotides.

In an alternative way of cloning a gene sequence which encodes the enzymes of the present invention, a library of expression vectors is prepared by cloning DNA or, more preferably, cDNA (from a cell capable of expressing the enzyme) into an expression vector. The library is then screened for members capable of expressing a protein which binds to enzyme-specific antibody, and which has a nucleotide sequence that is capable of encoding polypeptides that have the same amino acid sequence as the enzyme, or fragments thereof. In this embodiment, DNA, or more preferably cDNA, is extracted and purified from a cell which is capable of expressing the enzyme. The purified cDNA is fragmented (by shearing, endonuclease digestion, etc.) to produce a pool of DNA or cDNA fragments. DNA or cDNA fragments from this pool are then cloned into an expression vector in order to produce a genomic or cDNA library of expression vectors whose members each contain a unique DNA or cDNA fragment.

Thus, in summary, the identification of the substrate-specific aminopeptidases of the present invention permits the identification of a theoretical "most probable" DNA sequence, or a set of such sequences, capable of encoding peptide sequence of these enzymes. By constructing an oligonucleotide complementary to this theoretical sequence (or by constructing a set of oligonucleotides complementary to the set of "most probable" oligonucleotides), one obtains a DNA molecule (or set of DNA molecules), capable of functioning as a probe to identify and isolate a gene sequence capable of encoding any of the enzymes of the present invention.

Techniques such as, or similar to, those described above have successfully enabled the cloning of genes for human aldehyde dehydrogenases (Hsu, L.C., et al., Proc. Natl. Acad. Sci. USA 82:3771-3775 (1985)), fibronectin (Suzuki, S., et al., Eur. Mol. Biol. Organ. J. 4:2519-2524 (1985)), the human estrogen receptor gene (Walter, P., et al., Proc. Natl. Acad. Sci. USA 82:7889-7893 (1985)), tissue-type plasminogen activator (Pennica, D., et al., Nature 301:214-221 (1983)) and human term placental alkaline phosphatase complementary DNA (Kam, W., et al., Proc. Natl. Acad. Sci. USA 82:8715-8719 (1985)).

Using the above-described methods, oligonucleotide probes were constructed and employed to isolate a complete cDNA sequence capable of encoding the API aminopeptidase. The complete amino acid sequence of API, and the complete cDNA sequence capable of encoding the API aminopeptidase are presented in Figure 3. The protein was found to be synthesisized as a precursor molecule containing a signal sequence. This signal sequence is cleaved to produce the mature API aminopeptidase.

V. Expression of the Substrate-specific Aminopeptidase Encoding Sequences

DNA or cDNA molecules which encode the AP2, API or APX enzyme canbe operably linked into an expression vector and introduced into a host cell to enable the expression of the AP2, API or APX enzyme by that cell. Two DNA sequences (such as a promoter region sequence and a desired enzyme encoding sequence) are said to be operably linked if the nature of the linkage between the two DNA sequences does not (1) result in the introduction of a frame-shift mutation, (2) interfere with the ability of the promoter region sequence to direct the transcription of the desired enzyme encoding gene sequence, or (3) interfere with the ability of the desired enzyme gene sequence to be transcribed by the promoter region sequence.

A DNA sequence encoding AP2, API or APX may be recombined with vector DNA in accordance with conventional techniques, including blunt-ended or stagger-ended termini for ligation, restriction enzyme digestion to provide appropriate termini, filling in of cohesive ends as appropriate, alkaline phosphatase treatment to avoid undesirable joining, and ligation with appropriate ligases.

The present invention encompasses the expression of the desired enzyme in either prokaryotic or eukaryotic cells. Preferred eukaryotic hosts include yeast, fungi (especially Aspergillus), mammalian cells (such as, for example, human or primate cells) either in vivo, or in tissue culture.

Yeast are the preferred hosts of the present invention. The use of yeast provides substantial advantages in that yeast can also carry out post-translational peptide modifications including glycosylation. A number of recombinant DNA strategies exist which utilize strong promoter sequences and high copy number of plasmids which can be utilized for production of the desired proteins in yeast. Yeast recognize leader sequences on cloned mammalian gene products and secrete peptides bearing leader sequences (i.e., pre-peptides). Mammalian cells provide post-translational modifications to protein molecules including correct folding or glycosylation at correct sites.

Mammalian cells which may be useful as hosts include cells of fibroblast origin such as VERO or CHO-K1, and their derivatives. For a mammalian host, several possible vector systems are available for the expression of the desired enzyme. A wide variety of transcriptional and translational regulatory sequences may be employed, depending upon the nature of the host. The transcriptional and translational regulatory signals may be derived from viral sources, such as adenovirus, bovine papilloma virus, simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolite.

The expression of the desired enzyme in eukaryotic hosts requires the use of eukaryotic regulatory regions. Such regions will, in general, include a promoter region sufficient to direct the initiation of RNA synthesis. Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London 290:304-310 (1981)); the yeast gal4 gene promoter (Johnston, S.A., et al., Proc. Natl. Acad. Sci. (USA) 79:6971-6975 (1982); Silver, P.A. et al., Proc. Natl. Acad. Sci. (USA) 81:5951-5955 (1984)).

As is widely known, translation of eukaryotic mRNA is initiated at the codon which encodes the first methionine. For this reason, it is preferable to ensure that the linkage between a eukaryotic promoter and a DNA sequence which encodes the desired enzyme (or a functional derivative thereof) does not contain any intervening codons which are capable of encoding a methionine (i.e., AUG). The presence of such codons results either in the formation of a fusion protein (if the AUG codon is in the same reading frame as the desired enzyme encoding DNA sequence) or a frame-shift mutation (if the AUG codon is not in the same reading frame as the desired enzyme encoding sequence).

Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototrophic (ATCC 27325)), and other enterobacteria (such as Salmonella typhimurium or Serratia marcescens), and various Pseudomonas species. The prokaryotic host must be compatible with the replicon and control sequences in the expression plasmid.

To express the desired enzyme (or a functional derivative thereof) in a prokaryotic cell (such as, for example, E. coli, B. subtilis, Pseudomonas, Streptomyces, etc.), it is necessary to operably link the desired enzyme encoding sequence to a functional prokaryotic promoter. Such promoters may be either constitutive or, more preferably, regulatable (i.e., inducible or derepressible). Examples of constitutive promoters include the int promoter of bacteriophage λ, and the bla promoter of the β-lactamase gene of pBR322, etc. Examples of inducible prokaryotic promoters include the major left and right promoters of bacteriophage λ - (P$_L$ and P$_R$), the trp, recA, lacZ, lacI, gal, and tac promoters of E. coli, the α-amylase (Ulmanen, I., et al., J. Bacteriol. 162:176-182 (1985)), the σ-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell also requires the presence of a ribosome binding site upstream from the gene-encoding sequence. Such ribosome binding sites are disclosed, for example, by Gold, L., et al. (Ann. Rev. Microbiol. 35:365-404 (1981)).

The desired enzyme encoding sequence and an operably linked promoter may be introduced into a recipient prokaryotic or eukaryotic cell either as a non-replicating DNA (or RNA) molecule, which may either be a linear molecule or, more preferably, a closed covalent circular molecule. Since such molecules are incapable of autonomous replication, the expression of the desired enzyme may occur through the transient expression of the introduced sequence. Alternatively, permanent expression may occur through the integration of the introduced sequence into the host chromosome.

In one embodiment, a vector is employed which is capable of integrating the desired gene sequences into the host cell chromosome. Cells which have stably integrated the introduced DNA into their chromosomes can be selected by also introducing one or more markers which allow for selection of host cells which contain the expression vector. The marker may complement an auxotrophy in the host (such as leu2, or ura3, which are common yeast auxotrophic markers), biocide resistance, e.g., antibiotics, or heavy metals, such as copper, or the like. The selectable marker gene can either be directly linked to the DNA gene sequences to be expressed, or introduced into the same cell by co-transfection.

In a preferred embodiment, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Any of a series of yeast gene expression systems can be utilized. Examples of such expression vectors include the yeast 2-micron circle, the expression plasmids YEP13, YCP and YRP, etc., or their derivatives. Such plasmids are well known in the art (Botstein, D., et al., Miami Wntr. Symp. 19:265-274 (1982); Broach, J.R., In: The Molecular Biology of the Yeast Saccharomyces: Life Cycle and Inheritance, Cold Spring Harbor Laboratory, Cold Spring Harbor, NY, p. 445-470 (1981); Broach, J.R., Cell 28:203-204 (1982)). YEP13 is the preferred vector of the present invention.

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host chromosome. Cells which have stably integrated the introduced DNA into their chromosomes may be selected by also introducing one or more markers which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cell. Biol. 3:280 (1983), and others.

Preferred prokaryotic vectors include plasmids such as those capable of replication in E. coli (such as, for example, pBR322, ColE1, pSC101, pACYC 184, πVX. Such plasmids are, for example, disclosed by Maniatis, T., et al. (In: Molecular Cloning, A Laboratory Manual, Cole Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and Strptomyces bacteriophages such as φC31 (Chater, K.F., et al., In: Sixth International Symposium on Actinomycetales Biology, Akademiai Kaido, Budapest, Hungary (1986), pp. 45-54). Pseudomonas plasmids are reviewed by John, J.F., et al. (Rev. Infect. Dis. 8:693-704 (1986)), and Izaki, K. (Jpn. J. Bacteriol. 33:729-

14

742 (1978)).

Once the vector or DNA sequence containing the constructs has been prepared for expression, the DNA constructs may be introduced into an appropriate host. Various techniques may be employed, such as protoplast fusion, calcium phosphate precipitation, electroporation or other conventional techniques. After the fusion, the cells are grown in media and screened for appropriate activities. Expression of the desired sequence results in the production of the substrate-specific aminopeptidase.

The substrates-specific aminopeptidase of the invention may be isolated and purified from the above-described recombinant molecules in accordance with conventional methods, such as extraction, precipitation, chromatography, affinity chromatography, electrophoresis, or the like.

## VI. Uses of Substrate-Specific Aminopeptidase

Any of the substrate-specific aminopeptidases of the present invention, once produced and purified, can be used, for example, in a pharmaceutical, or manufacturing, environment to remove certain aminoterminal residues (consistent with each enzyme's substrate specificity as shown in Tables 2 and 3 below) from peptides and proteins for which such removal is desired in order to improve the stability, solubility, biological activity, biological half-life, etc. or to decrease the immunogenicity of the peptide or protein. It can, in addition, be used to cleave proteins, polypeptides, or peptides in accordance with its substrate specificities.

The substrate-specific aminopeptidase may be incubated with a desired substrate as a soluble enzyme, or may be immobilized, by means well known in the art, onto a solid support (such as, for example, glass, latex, plastic, cellulose, etc.)

Having now generally described this invention, the same will be better understood by reference to specific examples, which are included herein for purposes of illustration only, and are not intended to be limiting unless otherwise specified.

## EXAMPLE 1

## CELL GROWTH AND PREPARATION OF CRUDE EXTRACT

### Cell Growth and Storage

Yeast strain TD71.8 (alpha, ino3, leu2, his3, lys2) was grown at 30 °C in 1 liter YPD medium (1% yeast extract, 2% Bacto-peptone, 2% glucose) to $A_{600}$ ~18-20. The cells were harvested by centrifugation at 3,500 rpm for 10 min at 4 °C. The pellet (~25 g) was resuspended in 25 ml YPD medium containing 15% glycerol and stored at -70 °C.

For larger scale production and purification of enzyme, 1,000 liters of a culture of strain TD71.8 culture was grown aerobically at 30 °C in YPD medium (1% yeast extract, 2% Bacto-peptone, 2% glucose) in a Chemap AG fermentor (Chemap AG, Volketswil, Switzerland). Cells were harvested when the culture reached an $A_{600}$ of 8-10, concentrated to 38 liters by Alfa-Laval separation system (Alfa-Laval Separation AB, Tumba, Sweden), and stored at -20 °C with 10% (vol/vol) glycerol for up to 6 months without loss of activity.

### Preparation of Whole Cell Extracts

Frozen yeast cells (~500 g wet weight) were thawed in a 30 °C water bath and collected by centrifugation at 3,500 rpm for 10 minutes at room temperature. The pellet was resuspended in 1 liter of buffer S (1 M sorbitol, 50 mM Tris, pH 7.8, 10 mM $MgCl_2$, 3 mM DTT) containing 50 mg lyticase. This mixture was incubated at 30 °C for 2 hours with shaking to induce spheroplast formation. Spheroplasts were collected by centrifugation at 3,500 rpm for 10 minutes at 4 °C and washed by gentle resuspension in 1 liter of cold buffer S, collected by centrifugation at 3,500 rpm for 10 minutes and resuspended gently in 500 ml of buffer A (10 mM HEPES-K$^+$, pH 7.0, 1.5 mM MgCl$_2$, 10 mM KCl and 0.5 mM DTT). All the subsequent steps were carried out at 4 °C. The spheroplasts were lysed in this hypotonic buffer by 12-15 strokes with a tight-fitting pestle followed by 15-20 strokes with a loose-fitting pestle in a Dounce homogenizer and then the final KCl concentration was adjusted to 0.2 M by adding cold 2 M KCl solution. The lysate was stirred on ice for 30 minutes, and the debris was removed by centrifugation at 17,000 rpm for 30 minutes. Glycerol was added to 10% in all the buffers used in subsequent purification procedures to additionally stabilize the aminopeptidase.

The fractions were assayed for substrate-specific aminopeptidase activity as described above.

<u>EXAMPLE 2</u>

<u>PURIFICATION OF THE SUBSTRATE-SPECIFIC AMINOPEPTIDASE</u>

<u>First DEAE-Sepharose CL-6B Chromatography</u>

The crude lysate was concentrated to 100 ml by the use of a PM-10 ultrafiltration membrane and dialyzed three times against 2 liters of buffer H (10 mM HEPES-K$^+$, pH 7.35, 1.5 mM MgCl$_2$, 0.5 mM DTT, 0.2% NaN$_3$, 10% glycerol) supplemented to contain 0.05 M KCl. The lysate was then loaded on a CM-Sepharose CL-6B column (2.5 x 45 cm) which had been connected to a DEAE-Sepharose CL-6B column (2.5 x 45 cm) such that the eluate from the CM-Sepharose Cl-6B column was loaded continually to the connected DEAE-Sepharose CL-6B column. Both columns had been equilibrated with the same buffer as that used for dialysis. These two connected columns were washed with 2 liters of buffer H containing 0.05 M KCl. They were then disconnected. The DEAE-column was eluted with a linear gradient of 0.05 M (250 ml) to 0.5 M (250 ml) KCl in buffer H at 17.3 ml/hr. Fractions (5.2 ml) were collected and assayed for aminopeptidase activity. The fractions which contain aminopeptidase activity were pooled (fractions 41-71 of 143 total fractions; average conductivity 9.7 ms) and concentrated to a volume of 10 ml using a PM-10 ultrafiltration membrane.

<u>Second DEAE-Sepharose CL-6B Chromatography</u>

The concentrated eluate from the above-described DEAE-Sepharose CL-6B chromatography was dialyzed three times against 1 liter of buffer H supplemented to contain 0.05 M KCL and applied to a second DEAE-Sepharose CL-6B column (2.5 x 45 cm) equilibrated with the same buffer as that used for dialysis. The column was eluted with a linear gradient of 0.05 M (250 ml) to 0.2 M (250 ml) KCl in buffer H at 18 ml/hr. Fractions (4.5 ml) were collected and assayed for aminopeptidase activity. Fractions 113-133 were found to have AP2, API and APX activity. These fractions were pooled (average conductivity 10.2 ms). The pooled fractions were concentrated to 5 ml by a PM-10 ultrafiltration membrane.

<u>Hydroxylapatite Chromatography</u>

The concentrated eluate from the above-described DEAE-Sepharose CL-6B chromatography is dialyzed three times against 1 liter of potassium phosphate buffer, pH 7.15, 0.5 M DTT, 5 mM KCl, 10% glycerol, 0.02% NaN$_3$, and applied to a hydroxylapatite column equilibrated against the same buffer as that used for dialysis. The column was eluted with a linear gradient of 0.01 M (250 ml) to 0.5 M (250 ml) potassium phosphate buffer, pH 7.15, 0.5 mM DTT, 5 mM KCl, 10% glycerol, 0.02% NaN$_3$, at 18 ml/hr. Fractions (4.5 ml) were collected and assayed for aminopeptidase activity. Aminopeptidase AP2 was found in fractions 43-47 (average conductivity 7.2 ms). Aminopeptidase APX was found in fractions 67-75 (average conductivity 16.5 ms). Amino- peptidase API was found in fractions 83-97 (average conductivity 23.0 ms). Fractions containing each of the aminopeptidases were separately pooled, and concentrated to a volume of 5 ml using PM-10 ultrafiltration membrane.

## Phenyl-Sepharose CL-4B Chromatography

Aminopeptidase API was further purified using Phenyl-Sepharose CL-4B chromatography. The concentrated eluate from the above-described hydroxylapatite chromatography was dialyzed three times against 1 liter of buffer H (10 mM HEPES-K[+], pH 7.35, 0.5 mM DTT, 1.5 mM $MgCl_2$, 0.02% $NaN_3$, 10% glycerol) containing 0.2 M KCl. Equal volume of 80% saturated ammonium sulfate solution, 10 mM HEPES-K[+], pH 7.35, 10% glycerol, 0.02% $NaN_3$ were added to the solution containing aminopeptidase. This mixture was applied to a phenyl-Sepharose CL-6B column (1.2 x 20 cm) equilibrated with 40% saturated ammonium sulfate solution, 10 mM HEPES-K[+], pH 7.35, 10% glycerol, 0.25 mM DTT, 0.1 M KCl, 0.02% $NaN_3$. The column was first eluted with 50 ml of the same buffer as that described above and then with a linear gradient of 40% (100 ml) to 0% (100 ml) ammonium sulfate solution, 10 mM HEPES-K[+], pH 7.35, 10% glycerol, 0.25 mM DTT, 0.02% $NaN_3$, 200 mM KCl at 72.4 ml/hr. Fractions of 3.62 ml are collected and assayed for aminopeptidase activity. Aminopeptidase API was found in fractions 103-113 of 141 total fractions; the active fractions are pooled (average conductivity approximately 26 ms), and concentrated to 3 ml using a PM-10 ultrafiltration membrane.

The profiles of the fractions containing substrate-specific aminopeptidase activity are shown in Table 1.

Table 1

| Profile of Activity Containing Fractions | | | | |
|---|---|---|---|---|
| Column | Activity Data | | Protein Concentration 281 nm[*] | Average Conductivity of Active Fractions (ms) |
| | Fraction # | $A_{414}$ nm[*] | | |
| AP2 | | | | |
| 1st DEAE-Sepharose CL-6B | 41-71 | 0.293 | 2.817 | 9.7 |
| 2nd DEAE-Sepharose CL-6B | 113-133 | 0.38 | 2.66 | 10.2 |
| Hydroxylapatite | 43-47 | 0.30 | 2.25 | 7.2 |
| API | | | | |
| 1st DEAE-Sepharose CL-6B | 41-71 | 0.293 | 2.817 | 9.7 |
| 2nd DEAE-Sepharose CL-6B | 113-133 | 0.38 | 2.66 | 10.2 |
| Hydroxylapatite | 83-97 | 0.797 | 0.101 | 23.0 |
| Phenyl-Sepharose CL-4B | 103-113 | 0.36 | 0.123 | 26.0 |
| APX | | | | |
| 1st DEAE-Sepharose CL-6B | 41-71 | 0.293 | 2.817 | 9.7 |
| 2nd DEAE-Sepharose CL-6B | 113-133 | 0.38 | 2.66 | 10.2 |
| Hydroxylapatite | 67-75 | 0.39 | 0.232 | 16.5 |

[*] Absorbance is average of pooled active fractions

In an alternative method for purifying API, a crude yeast extract was obtained from 2 liters of concentrated cells according to the method of Line et al. (Lin, R.J. et al., J. Biol. Chem. 260:14780-14792 (1985)). The API present in such a sample was purified to near homogeneity by the method of Metz and Rohm (Metz, G. et al., Biochem. Biophys. Acta 429:933-949 (1976)). Aliquots of the concentrated eluate from the Sepharose CL-6B chromatography are preferably applied to three SDS-PAGE gels (8%) with a thickness of 1.5 mm and with a well size of 12 cm, electrophoresed, and electroeluted, as described by Hunkapiller, M.W. et al., (Methods Enzymol. 91:227-247 (1983)). When this alternative purification method was employed, the 2 liters of concentrated cells were found to contain approximately 900 $\mu$g of API. A purification of approximately 800-fold was achieved by this method and a yield of ~350 $\mu$g of API was realized.

Partially purified API, purified through the DEAE-Sepharose CL-6B chromatography step of Metz and Rohm (Metz, G. et al., Biochem. Biophys. Acta 429:933-949 (1976)) was applied to a Sepharose CL-6B column (2.0 x 90 cm). The column was eluted with B buffer (20 mM HEPES-K$^+$, pH 7.35, 5 mM MgCl$_2$, 0.5 nM DTT. 0.2 M KCl, 0.02% NaN$_3$) at 14 ml/hr. The elution volume of the enzyme was determined by A$_{280}$ nm and enzyme activity, and the apparent molecular weight of yeast API was calculated by comparison with the relative elution volumes of protein standards including thyroglobulin (669,000), apoferritin (443,000), $\beta$-amylase (200,000), alcohol dehydrogenase (150,000), bovine serum albumin (66,000), and carbonic anhydrase (29,000).

A sample of purified API was loaded on a SDS-Page gel (9%) and electrophoresed under reducing conditions, as described by Laemmli (Laemmli, U.K., Nature 227:680-685 (1970)). For determination of M$_r$ of purified enzyme subunits, myosin (205,000), E. coli $\beta$-galactosidase (116,000), rabbit muscle phosphorylase (97,000), bovine serum albumin (66,000), egg albumin (45,000) and carbonic anhydrase (29,000) were used as molecular weight standards. Protein bands were strained with Coomassie Brilliant Blue R. The native and subunit apparent molecular weights of API (purified according to this alternative method) were indistinguishable from the values obtained when API was purified according to the preffered method.

## EXAMPLE 3

## ESTIMATION OF THE MOLECULAR WEIGHT OF AMINOPEPTIDASE BY SDS-POLYACRYLAMIDE GEL ELECTROPHORESIS

Electrophoresis was performed according to the method of Laemmli, U.K. (Nature 227:680-685 (1970)) using an 10% gel. The gel was stained with Coomassie blue, and the apparent molecular weight of the denatured aminopeptidase was calculated from the relative mobility of protein standards including (1) myosin (205,000), (2) E. coli beta-galactosidase (116,000), (3) rabbit muscle phosphorylase (97,000), (4) bovine serum albumin (66,000) and (5) egg albumin (45,000), and (6) carbonic anhydrase (29,000). The apparent molecular weight of AP2 was approximately 93,000, and the apparent molecular weight of API was approximately 50,000.

## EXAMPLE 4

## SUBSTRATE-SPECIFICITY

Thirty-six peptides were made to study the substrate specificity of the purified aminopeptidases of the present invention. Eighteen of the peptides had different N-terminal amino acid residues, and the remaining eighteen peptides had different amino acid residues at the penultimate amino-terminal position. The protein solution (10 μl) to be assayed was added to 90 μl of the solution containing 4 mM M-X-I-P-E-T or X-I-P-E-T, 10 mM HEPES-K$^+$, pH 7.35, 0.2 mM CoCl$_2$, 0.2 M KCl, 1.5 mM MgCl$_2$, 0.02% NaN$_3$, 30 μg of L-amino acid oxidase, 3 μg of horseradish peroxidase and 20 μg of o-dianisidine dihydrochloride. All the reactions were carried out at room temperature on a 96-well microtiter plate, and absorbance detected at 414 or 450 nm. Rapid color development indicates the presence of high peptidase activity on that specific peptide. The results of these experiments are shown in Tables 2 and 3. The identity of X is designated by the commonly used single letter abbreviations for the common amino acids: V = Val, A = Ala, R = Arg, D = Asp, C = Cys, E = Glu, Q = Gln, G = Gly, H = His, I = Ile, L = Leu, K = Lys, M = Met, F = Phe, S = Ser, T = Thr, and W = Trp. The numbers in Tables 2 and 3 represent qualitative estimates of activity: 0 (no activity); 1 ( +/-); 2 (+); 3 (+ +/-); 4 (+ +); 5 (+ + +/-); and 6 (+ + +).

## Table 2

## Substate-specificity: M-X-I-P-E-T

| Identity of X: | V | A | R | D | C | E | Q | G | H | I | L | K | M | F | P | S | T | W |
|---|---|---|---|---|---|---|---|---|---|---|---|---|---|---|---|---|---|---|
| ENZYME: AP2 | 0 | 0 | 2 | 2 | 0 | 0 | 4 | 2 | 2 | 2 | 2 | 2 | 2 | 2 | 0 | 0 | 0 | 0 |
| API | 0 | 4 | 3 | 2 | 2 | 0 | 0 | 4 | 4 | 4 | 2 | 2 | 2 | 2 | 0 | 4 | 0 | 2 |
| APX | 1 | 1 | 2 | 1 | 2 | 2 | 1 | 4 | 2 | 2 | 2 | 2 | 2 | 2 | 2 | 1 | 2 | 2 |

## Table 3

## Substrate-specificity: X-I-P-E-T

| Identity of X: | V | A | R | D | C | E | Q | G | H | I | L | K | M | F | P | S | T | W |
|---|---|---|---|---|---|---|---|---|---|---|---|---|---|---|---|---|---|---|
| ENZYME: AP2 | 0 | 1 | 2 | 0 | 0 | 0 | 1 | 0 | 1 | 1 | 6 | 0 | 6 | 6 | 1 | 1 | 1 | 4 |
| API | 0 | 0 | 2 | 0 | 0 | 0 | 1 | 0 | 1 | 0 | 6 | 0 | 6 | 6 | 1 | 1 | 1 | 4 |
| APX | 1 | 1 | 2 | 0 | 0 | 0 | 1 | 1 | 1 | 1 | 6 | 0 | 6 | 6 | 0 | 0 | 1 | 2 |

## EXAMPLE 5

### PURIFICATION AND NH₂-TERMINAL PROTEIN SEQUENCE OF YEAST AMINOPEPTIDASE I

API aminopeptidase was purified from a cell lysate of log phase cells (i.e., $A_{600}$ = 8-10) as described above and used for the NH$_2$-terminal protein sequence. The subunit of the aminopeptidase was purified to homogeneity by SDS-PAGE and electrophoretically eluted (Hunkapiller, M.W. et al., Methods Enzymol. 91:227-247 (1983)), and its $M_r$ was estimated to be approximately 50,000 ± 2,000.

The amount of API and its amino acid composition was determined using a Beckman 6300 Amino Acid Analyzer after 24 h hydrolysis at 110°C in 6 N HCl containing 0.1% phenol (Moore, S., Chemistry and Biology of Peptides, Meienhofer, J., ed., Ann Arbor Science, Ann Arbor, MI, pp. 629-652 (1972)).

Five hundred pmoles of the purified API were analyzed using an Applied Biosystems 470A Protein Sequencer and an Applied Biosystems 120 PTH Analyzer (Hewick, R.M. et al., J. Biol. Chem. 256:7990-7997 (1981)). The average repetitive yield was approximately 92% and the initial amount of protein detected at degradative cycle 1 was approximately 250 pmol. The NH$_2$-terminal proteins sequence of purified API is shown in Figure 1.

When, however, the subunit was purified from a cell lysate obtained from a culture grown to stationary phase (i.e., $A^{600}$ = 18-20) instead of log phase, the NH$_2$-terminal sequence of API was found to begin at Tyr, the fourth amino acid residue from the NH$_2$-terminus.

## EXAMPLE 6

19

## CLONING AND SEQUENCING OF THE YEAST GENOMIC DNA ENCODIDNG AMINOPEPTIDASE I

Two degenerate probes were synthesized based on the N-terminal sequence of aminopeptidase I (Figure 1, probes a and b) and were used sequentially to screen a λgt11 yeast genomic library. The oligonucleotide probes and primers were synthesized on an Applied Biosystems 380A DNA synthesizer, using β-cyanoethyl phosphoramidites (Sinha, N.D. et al., Nucleic Acids Res. 12:4539-4557 (1984)) and purified by polyacrylamide gel electrophoresis according to the Applied Biosystems Manual or by ethanol precipitation from a solution of oligonucleotide containing 10 mM $MgCl_2$.

A λgt11 yeast genomic library was screened sequentially using two [$^{32}$P]-labeled oligonucleotide probes (Figure 1, probes a and b). Phage plaques were transferred onto nitrocellulose filters and lysed (Maniatis, T. et al., Molecular Cloning, A Laboratory Manual, Cold Spring Harbor Laboratory, Cold Spring Harbor, New York (1982)). The filters were baked for 2 h at 80°C and then incubated at 65°C for 3 h in 2 x 1 liter of washing buffer (0.2xSSC, 0.1% SDS). The washed filters were incubated in prehybridization buffer (6xSSC, 5x Denhardt's solution, 0.5% SDS, 0.05% sodium pyrophosphate, 100 μg/ml yeast t-RNA, and 100 μg/ml sheared and denatured herring sperm DNA) for 12 to 16 h at 65°C. Finally, hybridization was performed at 55°C overnight using the same buffer in the presence of the degenerate oligonucleotides labeled with [γ-$^{32}$P]ATP by T4 polynucleotide kinase (Whitehead A.S. et al., Proc. Natl. Acad. Sci. USA 80:31-35 (1983)). Nitrocellulose filters were washed at room temperature for 30 min in 6xSSC before washing at 55°C (high stringency) for 10 min. After the final wash, the filter was exposed to Kodak XAR-5 film at -70°C.

Seventeen positive clones were obtained from ~100,000 plaques. Three clones were further analyzed by restriction mapping and shown to be identical. One of the inserts was subcloned into Bluescript M13+, and a portion of the DNA sequence was determined using a T7 sequencing primer. This sequence corresponds to a sequence encoding the $NH_2$-terminal sequence of the purified API, shown in Figure 1. Based on DNA sequence adjacent to the T7 promoter an additional oligonucleotide, probe c (5′ CGAGCACAATTATGAGGATATTGC 3′), was synthesized and used to screen an EMBL3A yeast genomic library. Forty-seven positive clones were obtained from ~50,000 plaques, and three of these were analyzed by restriction mapping. A Sall restriction fragment (~14kb) from one of these clones was subcloned into Bluescript M13+, and its partial restriction map is shown in Figure 2. The PstI, XbaI, XbaI/PstI, PstI/EcoRI, EcoRI/XbaI, and EcoRI restriction fragments were subcloned into Bluescript M13+. DNA sequences were obtained from the 5′- and 3′-ends of those subclones. Based on those DNA sequences, additional oligonucleotide primers were synthesized and used to complete the sequence of both DNA strands.

## EXAMPLE 7

## DNA SEQUENCE AND DERIVED AMINO ACID SEQUENCE OF AMINOPEPTIDASE I

Nucleotide sequence analysis was carried out by subcloning restriction fragments into Bluescript M13+ vector, which contains T3 and T7 promoters. After alkaline denaturation of Bluescript M13+ containing fragments of the cloned gene, T3 or T7 primers were used to prime DNA synthesis in the presence of dideoxynucleotide triphosphates (Sanger F. et al., Proc. Natl. Acad. Sci. Usa 74:5463-5467 (1977); Sanger, F. et al., J. Mol. Biol. 143:161-178 (1980)). After obtaining portions of the DNA sequence, homologous 17-base oligonucleotide primers were synthesized for additional DNA sequencing using the dideoxy chain termination method of Sander (Sanger, F. et al., Proc. Natl. Acad. Sci. USA 74:5463-5467 (1977); Sanger, F et al., J. Mol. Biol. 143:161-178 (1980)), modified for double-stranded sequencing by Guo et al. (Guo, L.H. et al., Nucleic Acids Res. 11:5521-5539 (1983)). The complete DNA sequence of both strands was determined. Figure 3 shows the complete DNA sequence and deduced protein sequence for yeast API.

The most 5′ ATG, which obeys Kozak's rule (Kozak, M., Nucleic Acids Res. 12:857-872 (1984)) is presumed to be the initiation codon. An open reading frame of 1542 nucleotides (encoding aminopeptidase I and its presequence) extends from this initiation codon ATG to a stop codon ACT (marked End). The first 105 nucleotides beginning with the initiation codon encodes a presequence of 45 amino acids, beginning with a Met at residue -45. The open reading frame sequence encodes a protein with 514 amino acid residues and with a calculated molecular weight of 57,175 daltons. The $NH_2$-terminal sequence derived from protein sequence analysis of the first 18 amino acid residues of the purified API and matches residues 1-18 of API deduced from the DNA sequence. This data is supported by immuoblot data and indicates that there is an API precursor, containing a 45 amino acid residue presequence, which is removed during maturation of the enzyme.

A TATA-like sequence, TATAAG, is located 102 bases upstream of the initiation codon. CACAACCAACA, which is located within 20 to 30 nucleotides of the initiation codon, may be a ribosomal-binding site (Montgomery, D.L. et al., Proc. Natl. Acad. Sci. USA 77:541-545 (1980); Holland, M.J. et al., J. Biol. Chem. 256:1385-1395 (1981)). Furthermore, 150 bases downstream from the stop codon, there is a putative polyadenylation signal, AAATAAA (Proudfoot, N.J. et al., Nature 26: 211-214 (1976)). In addition, there are four potential N-glycosylation sites (Parodi, A.J. et al., Biochem. Biophys. Acta 559:1-37 (1979)), indicated by asterisks.

## EXAMPLE 8

## PREPARATION AND AFFINITY PURIFICATION OF POLYCLONAL ANTIBODIES AGAINST YEAST AMINOPEPTIDASE I

SDS-PAGE purified, electroeluted aminopeptidase I (50 $\mu$g) was injected intradermally in complete Freund's adjuvant into a New Zealand white rabbit and followed by two injections (biweekly) of 50 and 100 $\mu$g, respectively. Ten days after the final immunization, the animal was bled. Immunoglobulin was precipitated from serum (20 ml) by the addition of $(NH_4)_2SO_4$ to 50% saturation, followed by centrifugation at 10,000 x g for 20 min. The antibody pellet (~50 mg) was resuspended in a minimal amount of phosphate-buffered saline (PBS; 0.15 M NaCl, 12.5 mM $KH_2PO_4$, pH 7.4, 0.02% $NaN_3$) and dialysed against PBS.

The antibodies were further purified by an affinity column (bed volume: 1 ml) containing 100 $\mu$g of SDS-PAGE purified and electroeluted API covalently attached to CNBr-activated Sepharose 4B (Pharmacia), which was prepared according to the manufacturer's instructions. One milliliter of the antibody solution was passed repeatedly through the affinity column at room temperature. Then the column was washed with 0.6 M KCl in PBS, until the $A_{280}$ of the eluate was <0.005. The bound antibodies were eluted with 2.5 ml of 150 mM NaCl, 0.02% $NaN_3$ and 0.2 M glycine, pH 2.4, and the eluate was neutralized immediately with 1 N NaOH. Antibodies (~75 $\mu$g) were recovered and used for immunoblot analysis.

## EXAMPLE 9

## IMMUNODETECTION OF PURIFIED AMINOPEPTIDASE I AND TOTAL YEAST PROTEIN

SDS-PAGE purified and electroeluted API was analyzed by immunoblot. To accomplish this, purified aminopeptidase I and the extract of total yeast protein wer electrophoresed on a SDS-PAGE gel (8%) (Laemmli, U.K., Nature 227:680-685 (1970)) and then electrophoretically transferred to a nitrocellulose filter (Towbin, H. et al., Proc. Natl. acad. Sci. USA 76:4350-4354 (1979)).

A 20 ml culture of yeast strain TD71.8 was grown at 30° C to an $A_{600}$ of 8 to 10. Pelleted cells (0.2-0.3 g) were collected at 3,000 x g for 5 min, resuspended in 3 ml of A buffer (10 mM Tris, pH 7.5, 1 M sorbitol, 5 mM $MgCl_2$, 3 mM DTT) containing 0.1 mg/ml of lyticase and then incubated at 30° C for 30 min. The spherophasts were collected by centrifugation at 1,000 x g for 3 min and resuspended in 0.8 ml of B buffer containing 0.3 g of glass beads (0.5 mm dia.) and transferred to a 2 ml microfuge tube. Cells were broken by 6 cycles of vortexing for 30 sec followed by sitting on ice for 30 sec. An equal volume of 2X SDS sample buffer (20% glycerol, 5% mercaptoethanol, 4% SDS, 0.13 M Tris, pH 6,8, 0.01%, (vol/vol) bromophenol blue) was added and boiled for 10 min. The non-dissolved material was removed by microcentrifugation for 3 min at room temperature. Supernatant (100 $\mu$l), as well as protein molecular weight markers, were electrophoresed on an 8% SDS-PAGE gel (Laemmli, U.K., Nature 227:680-685 (1970)). Proteins were electrophoretically transferred to a nitrocellulose membrane (Towbin, H. et al., Proc. Natl. Acad. Sci. USA 76:4350-4354 (1979)).

In order to detect aminopeptidase I and its precursor, the membrane containing transferred proteins was reacted with affinity purified anti-aminopeptidase I rabbit polyclonal antibody, and the bound antibody was detected with a picoBlue immunodetection kit (Stratagene), according to the supplier's instructions. The apparent molecular weights were calculated using protein standards including rabbit muscle phosphorylase (97,000), bovine serum albumin (66,000), egg albumin (45,000), and carbonic anhydrase (29,000).

Immunoblot analysis of purified API detected a single protein of $M_r$ = 50,000 that binds rabbit polyclonal anti-API antibody, and immunoblot analysis of an extract of total yeast protein detects two proteins with $M_r$'s of 50,000 and 57,000, respectively.


## EXAMPLE 10


## GENOMIC AND CHROMOSOMAL DNA BLOT ANALYSIS


Genomic and chromosomal DNA blot analyses were performed. To accomplish this, total DNA was isolated from yeast strain TD71.8 (Sherman, F. et al., Methods in Yeast Genetics, A Laboratory Manual, Cold Spring Harbor Laboratory, Cold Spring Harbor, New York (1986)). DNA (10 µg) was digested with EcoRI or XbaI and electrophoresed on a 0.7% agarose gel. Transfer of the digested DNA fragments from the gel to a nitrocellulose paper was performed according to the method of Southern (Southern, E.M., J. Mol. Biol. 98:503-517 (1975)). The filter was rinsed with 6xSSC for 5 min before being baked at 80°C for 2 h. The baked filter was incubated at 65°C for 3 h in the washing buffer (0.1% SDS, 0.2xSSC). The filter was then prehybridized in 6xSSC, 0.05% sodium pyrophosphate, 0.5% SDS, 100 µg/ml yeast t-RNA, 5x Denhardt's solution, and 100 µg/ml sheared and denatured herring sperm DNA for 12 to 16 h at 65°C. Hybridization was performed at 65°C overnight using the same buffer in the presence of nick-translated (Rigby, P.W.J. et al., J. Mol. Biol. 113:237-251 (1977)) PstI restriction fragment (~2 kb) (Figure 2). The filter was washed twice for 15 min with 6xSSC, 0.1% SDS, and 0.05% sodium pyrophosphate at room temperature and then washed twice at 65°C for 10 min in 0.2xSSC, 0.1% SDS. After the final wash, the filter was exposed to Kodak XAR-5 film at -70°C.

The yeast chromosomal agarose gel (i.e., a "Saccharomyces chromo-di-hybridizer" from Clontech) was probed with the [$^{32}$P]-labeled PstI restriction fragment (~2 kb) of the yeast aminopeptidase I gene (Southern, E.M., J. Mol. Biol. 98:503-517 (1975); Rigby, P.W.J. et al., J. Mol. Biol. 113:237-251 (1977)) (Figure 2), according to the supplier's instructions. After the final wash, the dried gel was exposed to Kodak XAR-5 film at -70°C.

Three major bands were detected in the EcoR1 digested genomic DNA blot, corresponding to EcoRI restriction fragments of about 3.8 kb, 0.9 kb and 0.5 kb. Two major bands were detected in the XbaI digested genomic DNA blot, corresponding to XbaI restriction fragments of about 2.7 kb and 2.5 kb. These results were consistent with those predicted from the restriction map of the cloned gene (Figure 2). The sizes of the two small EcoRI restriction fragments (deduced from the DNA sequence in Figure 3) were expected to be 920 bp and 445 bp, which agreed with the data obtained. The additional fragment at 3.8 kb was predicted from the restriction map (Figure 2), but its size could not be precisely calculated. The 2.7 kb XbaI fragment was predicted from the restriction map (Figure 2). The additional fragment at 2.5 kb was also predicted from the restriction map (Figure 2), but again its size could not be precisely calculated. These data indicate that there is a single copy of the gene encoding API contained in the yeast genome.

DNA blot analysis of yeast chromosomal DNA showed that the API gene is located on yeast chromosome XI.


## EXAMPLE 11


## STRUCTURE OF THE PRESEQUENCE OF YEAST AMINOPEPTIDASE I


Figure 4 shows the NH$_2$-terminal residues -45 to -27 of the API precursor protein displayed in a helical wheel (Schiffer, M. et al., Biophys. J. 7:121-135 (1967)). Residues -45 to -42 are not arrayed in the α-helix. The presence of both a hydrophobic (consisting of primarily of Leu and Ile residues) and a hydrophilic (consisting of Lys, Arg, Glu, Gln, and Met residues) face in this predicted α-helix is characteristic of an amphiphilic α-helix (Segrest, J.P,. et al., FEBS Letters 38:247-258 (1974)).

The computer program FASTP (Lipman, D.J. et al., Science 227:1435-1331 (1985)) was used to compare the derived amino acid sequence of yeast API with the entire National Biomedical Research Foundation protein sequence data bank, and no significant sequence similarity with any other proteins was found. The GAP program (Needleman, J.B. et al., J. Mol. Biol. 48:443-453 (1970)) was used to compare the sequence of API with bovine leucine aminopeptidase (Cuypers, H.T. et al., J. Biol. Chem. 257:7077-7085 (1982)), E. coli peptidase N (McCaman, M.T. et al., Mol. Gen. Genet. 204:148-152 (1986)) and E. coli methionine-specific aminopeptidase (Ben-Bassat, A. et al., J. Bacteriol. 169:751-757 (1987)), and no significant sequence homology was observed.

## EXAMPLE 12

## CHARACTERISTICS OF AMINOPEPTIDASE API

Yeast aminopeptidase I is a vacuolar enzyme, which catalyzes the removal of amino acids from the $NH_2$-terminus of peptides and proteins (Frey, J. et al., Biochem. Biophys. Acta 527:31-41 (1978)). The $NH_2$-terminal sequence for the first 18 residues of SDS-PAGE purified aminopeptidase I was determined, and two synthetic oligonucleotide probes based on this protein sequence were synthesized and used to screen a yeast λgt11 genomic library. Using a probe derived from a sequence of one of isolated λgt11 inserts, yeast genomic DNA encoding aminopeptidase I was cloned from a yeast EMBL3A library and sequenced. The DNA sequence encodes a precursor protein containing 514 amino acid residues. The "mature'" protein, whose $NH_2$-terminal sequence was confirmed by automated Edman degradation, consists, based only on the DNA sequence, of 469 amino acids. A 45 residue presequence contains positively and negatively charged, as well as hydrophobic residues, and its $NH_2$ residues could be arranged in an amphiphilic α-helix. This presequence differs from the signal sequences which direct proteins across bacterial plasma membranes and endoplasmic reticulum or into mitochondria. This unique presequence, with its mixture of charged (positively and negatively) and hydrophobic residues, targets aminopeptidase I to yeast vacuoles. Further, the aminopeptidase I gene, localized previously by genetic mapping to yeast chromosome XI and called the lap4 gene (Trumbly, R.J. et al., J. Bacteriol. 156:36-48 (1983)), was determined by DNA blot analyses to be a single copy gene located on chromosome XI.

The $M_r$ of the native enzyme (570,000) and its subunit (50,000) agree with the results of others, who determined that the protein was a multimeric glycoprotein (Johnson, M.J., J. Biol. Chem. 137:575-586 (1941); Trumbly, R.J. et al., J. Bacteriol. 156:36-48 (1983); Frey, J. et al., Biochem. Biophys. Acta 527:31-41 (1978); Metz, G. et al., Biochem. Biophys. Acta 429:933-949 (1976)).

## EXAMPLE 13

## CHARACTERIZATION OF THE CLONED API GENE SEQUENCE

The cloned genomic API DNA contains a TATA-like sequence upstream of the initiation codon and a polyadenylation signal downstream of the stop codon (Figure 3). The genomic and chromosomal DNA blot analyses indicate that there is a single copy of the API gene located on yeast chromosome XI, which is consistent with previous genetic mapping of the lap4 gene by Trumbly and Bradley (Trumbly, R.J. et al., J. Bacteriol. 156:36-48 (1983)).

The open reading frame of the genomic DNA contains 1542 nucleotides encoding an API precursor containing 514 amino acid residues. The calculated molecular weight of the precursor is 57,175 daltons, which agrees with $M_r$ of the precursor estimated by immunoblot analysis. A comparison of this deduced protein sequence with the N-terminal sequence of the purified API indicated that the purified protein lacks 45 residues at the $NH_2$-terminus of the precursor protein. Further, the calculated molecular weight of the processed precursor protein (lacking its $NH_2$-terminal 45 amino acid residues) is 51.663, which agrees with the $M_r$ of the purified enzyme estimated by SDS-PAGE and immunoblot analyses. However, Metz and Rohm (Metz, G. et al., Biochem. Biophys. Acta 429:933-949 (1976)) have determined that API has a $M_r$ of 51.000 (including 12% carbohydrate) and calculated from amino acid analysis that the protein lacking carbohydrate had a estimated molecular weight of 44,800. If this is the case, then additional C-terminal processing must occur in order to generate the "mature" protein, described by Metz and Rohm (Metz, G. et al., Biochem, Biophys.. Acta 429:933-949 (1976)). How and where such processing occurs and whether or not it is mediated by vacuolar proteases, as has been demonstrated for the maturation of yeast proteinase B (Moehle. C.M. et al., Mol. Cell. Biol. 7:4390-4399 (1987)), remains to be established.

In addition, it was observed that if the protein was purified from yeast cells grown to stationary phase, rather to log phase as was the case for the protein whose $NH_2$-terminal sequence is shown in Figure 1, that the N-terminal sequence began at the Tyr, located at residue 4, but otherwise matched that deduced protein sequence.

Furthermore, the $NH_2$-terminus of the presequence contains a stretch of charged (positively and negatively) and hydrophobic residues capable of adopting an amphiphilic α-helical conformation (Figure 4), which is not typical of the presequences associated with other yeast vacuolar proteins, including proteinase A (Ammerer, g. et al., Mol. Cell. Biol. 6:2490-2499 (1986)), proteinase B (Moehle, C.M. et al., Mol. Cell. Biol. 7:4390-4399 (1987)), and CPY (Hasilik, A. et al., Eur. J. Biochem. 85:599-608 (1978)), and other eukaryotic secretory .proteins (for a review, see Briggs, M.S. et al., Adv. Prot. Chem. 38:109-180 (1986)). Their presequences are composed of a stretch of apolar amino acid residues, which are responsible for their targeting to endoplasmic reticulum (for a review, see Blobel, G. et al., In Biomembranes, Vol. 2, Manson, L.A., ed., Plenum, New York, pp. 193-195 (1971)).

The API presequence also differs from mitochondrial presequences, which are amphiphilic α-helices primarily containing positively charged residues (for a review, see Roise, D. et al., J. Biol. Chem. 263:4509-4511 (1988)). In addition although the prosequence of yeast CPY has been demonstrated to target CPY to the yeast vacuole (Johnson, L.M. et al., Cell 48:875-885 (1987); Valls, L.A. et al., Cell 48:87-897 (1987)), there is no obvious equivalent sequence responsible for the sorting of API into vacuoles.

The elements of the presequence of API responsible for its intracellular sorting may be evaluated using fusion proteins.

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

## Claims

1. The substantially purified substrate-specific aminopeptidase enzyme AP2 having an ability to cleave the peptide bond between an amino-terminal methionine residue, and every penultimate amino-terminal amino acid residue selected from the group consisting of: Arg, Asp, Gln, Gly, His, Ile, Leu, Lys, Met and Phe, and having an ability to cleave the peptide bond between an amino-terminal residue X, and an adjacent Ile residue of a peptide, wherein X is an amino acid residue selected from the group consisting of: Ala, Arg, Gln, His, Ile, Leu, Met, Phe, Pro, Ser, Thr and Trp.

2. The substantially purified substrate-specific aminopeptidase enzyme API having an ability to cleave the peptide bond between an amino-terminal methionine residue, and every penultimate amino-terminal amino acid residue selected from the group consisting of: Ala, Arg, Asp, Cys, Gly, His, Ile, Leu, Lys, Met, Phe, Ser and Trp, and having an ability to cleave the peptide bond between an amino-terminal residue X, and an adjacent Ile residue of a peptide, wherein X is an amino acid residue selected from the group consisting of: Arg, Gln, His, Leu, Met, Phe, Pro, Ser, Thr and Trp.

3. Substantially purified aminopeptidase API which contains at least one polypeptide selected from the group consisting of:

    a. Glu-His-Asn-Tyr-Glu-Asp-Ile-Ala-Gln-Glu-Phe-Ile-Asp-Phe-Ile-Tyr-Lys-Asn;

    b. Ser-Glu-Lys-Ser-Asn-Trp-Gln-Asp-Ser-Ile-Gly-Glu-Asp-Gly-Gly-Lys-Phe-Tyr;

    c. Val-Gly-Val-Ile-Gly-Ser-His-Val-Asp-Ala-Leu-Thr-Val-Lys-Leu-Lys-Pro-Val;

    d. Glu-Leu-Trp-Leu-Asp-Arg-Asp-Leu-Gly-Ile-Gly-Gly-Arg-Leu-Leu-Tyr-Lys-Lys;

    e. Ser-Leu-Ala-Pro-His-Phe-Gly-Lys-Pro-Ala-Glu-Gly-Pro-Phe-Asp-Lys-Glu-Asp;

f. Glu-Lys-Lys-Ser-Pro-Leu-Phe-Gly-Lys-His-Cys-Ile-His-Leu-Leu-Arg-Tyr-Val;

g. Lys-Gly-Thr-Ile-Gly-Gly-Ile-Gly-Lys-His-Phe-Leu-Phe-Ala-Pro-Arg-Leu-Asp;

h. Glu-Ser-Asp-Leu-Phe-Ser-Thr-Val-Thr-Leu-Tyr-Asp-Asn-Glu-Glu-Ile-Gly-Ser;

i. Thr-Lys-Lys-Pro-Val-Asp-Leu-His-Thr-Val-Trp-Ala-Asn-Ser-Ile-Ile-Leu-Ser;

j. Pro-Asn-Val-Gly-Ile-Thr-Leu-Ser-Leu-Asp-Pro-Asn-Gly-His-Met-Ala-Thr-Asp;

k. Ile-Lys-Asn-Asn-Ser-Arg-Ser-Gly-Gly-Thr-Ile-Gly-Pro-Ser-Leu-Ala-Ser-Gln; and

l. Gly-Ser-Lys-Asp-Val-Gly-Leu-Gly-Val-Lys-Phe-Phe-Asn-Gly-Phe-Phe-Lys-His.

4. A vacuolar localization presequence comprising the polypeptide:

Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu, optionally containing additionally an amino-terminal methionine residue.

5. The vacuolar localization presequence of claim 4, which is linked via a peptide bond to the amino-terminus of a heterologous protein or polypeptide.

6. The aminopeptidase API of claim 3 which is linked to a vacuolar localization presequence selected from the group consisting of:

a. Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu; and

b. Met-Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu.

7. The substantially purified substrate-specific aminopeptidase enzyme APX having an ability to cleave the peptide bond between an amino-terminal methionine residue, and every penultimate amino-terminal amino acid residue selected from the group consisting of: Val, Ala, Arg, Asp, Cys, Glu, Gln, Gly, His, Ile, Leu, Lys, Met, Phe, Pro, Ser, Thr and Trp, and having an ability to cleave the peptide bond between an amino-terminal residue X, and an adjacent Ile residue of a peptide, wherein X is an amino acid residue selected from the group consisting of: Val, Ala, Arg, Gln, Gly, His, Ile, Leu, Met, Phe, Thr and Trp.

8. A recombinant DNA molecule comprising a genetic sequence capable of encoding the substrate-specific aminopeptidases of claims 1, 2, 3 or 7.

9. A recombinant DNA molecule comprising a genetic sequence capable of encoding the vacuolor localization presequence of claim 4.

10. A recombinant DNA molecule comprising a genetic sequence capable of encoding the heterologous protein or polypeptide of claim 5.

11. A recombinant DNA molecule comprising a genetic sequence capable of encoding the substrate-specific aminopeptidase of claim 6.

12. The substrate-specific aminopeptidases AP2, API or APX of claims 1, 2, 3 or 7, which are produced by the expression of a recombinant molecule.

13. The heterologous protein or polypeptide of claim 5 which is produced by the expression of a recombinant molecule.

14. A method for recovering the substantially purified substrate-specific aminopeptidase of claim 1 from a sample containing said aminopeptidase comprising the following steps:

(a) recovering crude substrate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by said ion exchange chromatography, and, optionally:

(d) subjecting said recovered substrate-specific aminopeptidase of step (c) to hydroxylapatite chromatography; and

(e) recovering any substrate-specific aminopeptidase purified by said hydroxylapatite chromatography.

15. A method for recovering the substantially purified substrate-specific aminopeptidase of claim 2 from a sample containing said aminopeptidase comprising the following steps:

(a) recovering crude substrate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by said ion exchange chromatography, and optionally:

(d) subjecting said recovered substrate-specific aminopeptidase of step (c) to hydroxylapatite chromatography; and

(e) recovering any substrate-specific aminopeptidase purified by said hydroxylapatite chromatogrpahy, and, further optionally;

(f) subjecting said recovered substrate-specific aminopeptidase of step (e) to Phenyl-Sepharose CL-4B chromatography; and

(g) recovering any substrate-specific aminopeptidase purified by said Phenyl-Sepharose CL-4B chromatography.

16. A method for recovering the substantially purified substrate-specific aminopeptidase of claim 7 from a sample containing said aminopeptidase comprising the following steps:

(a) recovering crude substrate-specific aminopeptidase from a sample containing said aminopeptidase;

(b) subjecting said crude substrate-specific aminopeptidase from step (a) to ion exchange chromatogrpahy; and

(c) recovering any substrate-specific aminopeptidase purified by said ion exchange chromatography, and optionally:

(d) subjecting said recovered substrate-specific aminopeptidase of step (c) to hydroxylapatite chromatography; and

(e) recovering any substrate-specific aminopeptidase purified by said hydroxylapatite chromatography.

17. A method for removing an amino-terminal methionine residue from a peptide containing said amino-terminal residue, and containing as a penultimate amino-terminal amino acid residue, an amino acid selected from the group consisting of Arg, Asp, Gln, Gly, His, Ile, Leu, Lys, Met, and Phe, which method comprises incubating said peptide in the presence of the substrate-specific aminopeptidase AP2 to thereby remove said amino-terminal residue.

18. A method for removing an amino-terminal methionine residue from a peptide containing said amino-terminal residue, and containing as a penultimate amino-terminal amino acid residue, an amino acid selected from the group consisting of Ala, Arg, Asp, Cys, Gly, His, Ile, Leu, Lys, Met, Phe, Ser and Trp, which method comprises incubating said peptide in the presence of the substrate-specific aminopeptidase API to thereby remove said amino-terminal residue.

19. A method for removing an amino-terminal methionine residue from a peptide containing said amino-terminal residue, and containing as a penultimate amino-terminal amino acid residue, an amino acid selected from the group consisting of Val, Ala, Arg, Asp, Cys, Glu, Gln, Gly, His, Ile, Leu, Lys, Met, Phe, Pro, Ser, Thr and Trp, which method comprises incubating said peptide in the presence of the substrate-specific aminopeptidase APX to thereby remove said amino-terminal residue.

20. A method for removing an amino-terminal residue X from a peptide containing said terminal residue X, and an adjacent Ile residue, wherein X is selected from the group consisting of Ala, Arg, Gln, His, Ile, Leu, Met, Phe, Pro, Ser, Thr and Trp, which method comprises incubating said peptide in the presence of the substrate-specific aminopeptidase AP2 to thereby remove said amino-terminal residue.

21. A method for removing an amino-terminal residue X from a peptide containing said terminal residue X, and an adjacent Ile residue, wherein X is selected from the group consisting of Arg, Gln, His, Leu, Met, Phe, Pro, Ser, Thr and Trp, which method comprises incubating said peptide in the presence of the substrate-specific aminopeptidase API to thereby remove said amino-terminal residue.

22. A method for removing an amino-terminal residue X from a peptide containing said terminal residue X, and an adjacent Ile residue, wherein X is selected from the group consisting of Val, Ala, Arg, Gln, Gly, His, Ile, Leu, Met, Phe, Thr and Trp, which method comprises incubating said peptide in the presence of the substrate-specific aminopeptidase APX to thereby remove said amino-terminal residue.

23. The method of any one of the claims 17 to 22, wherein said substrate-specific aminopeptidase is immobilized to a solid support.

Claims for the following contracting state: ES

1. A method for recovering the substantially purified substrate-specific aminopeptidase AP2 having an ability to cleave the peptide bond between an amino-terminal methionine residue, and every penultimate amino-terminal amino acid residue selected from the group consisting of Arg, Asp, Gln, Gly, His, Ile, Leu, Lys, Met and Phe, and having an ability to cleave the peptide bond between an amino-terminal residue X, and an adjacent Ile residue of a peptide, wherein X is an amino acid residue selected from the group consisting of: Ala, Arg, Gln, His, Ile, Leu, Met, Phe, Pro, Ser, Thr and Trp, from a sample containing said aminopeptidase comprising the following steps:

(a) recovering crude substrate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by said ion exchange chromatogrpahy, and, optionally:

(d) subjecting said recovered substrate-specific aminopeptidase of step (c) to hydroxylapatite chromatography; and

(e) recovering any substrate-specific aminopeptidase purified by said hydroxylapatite chromatography.

2. A method for recovering the substantially purified substrate-specific aminopeptidase API having an ability to cleave the peptide bond between an amino-terminal methionine residue, and every penultimate amino-terminal amino acid residue selected from the group consisting of Ala, Arg, Asp, Cys, Gly, His, Ile, Leu, Lys, Met, Phe, Ser and Trp, and having an ability to cleave the peptide bond between an amino-terminal residue X, and an adjacent Ile residue of a peptide, wherein X is an amino acid residue selected from the group consisting of: Arg, Gln, His, Leu, Met, Phe, Pro, Ser, Thr and Trp, or which contains at least one polypeptide selected from the group consisting of:

a. Glu-His-Asn-Tye-Glu-Asp-Ile-Ala-Gln-Glu-Phe-Ile-Asp-Phe-Ile-Tyr-Lys-Asn;

b. Ser-Glu-Lys-Ser-Asn-Trp-Gln-Asp-Ser-Ile-Gly-Glu-Asp-Gly-Gly-Lys-Phe-Tyr;

c. Val-Gly-Val-Ile-Gly-Ser-His-Val-Asp-Ala-Leu-Thr-Val-Lys-Leu-Lys-Pro-Val;

d. Glu-Leu-Trp-Leu-Asp-Arg-Asp-Leu-Gly-Ile-Gly-Gly-Arg-Leu-Leu-Tyr-Lys-Lys;

e. Ser-Leu-Ala-Pro-His-Phe-Gly-Lys-Pro-Ala-Glu-Gly-Pro-Phe-Asp-Lys-Glu-Asp;

f. Glu-Lys-Lys-Ser-Pro-Leu-Phe-Gly-Lys-His-Cys-Ile-His-Leu-Leu-Arg-Tyr-Val;

g. Lys-Gly-Thr-Ile-Gly-Gly-Ile-Gly-Lys-His-Phe-Leu-Phe-Ala-Pro-Arg-Leu-Asp;

h. Glu-Ser-Asp-Leu-Phe-Ser-Thr-Val-Thr-Leu-Tyr-Asp-Asn-Glu-Glu-Ile-Gly-Ser;

i. Thr-Lys-Lys-Pro-Val-Asp-Leu-His-Thr-Val-Trp-Ala-Asn-Ser-Ile-Ile-Leu-Ser;

j. Pro-Asn-Val-Gly-Ile-Thr-Leu-Ser-Leu-Asp-Pro-Asn-Gly-His-Met-Ala-Thr-Asp;

k. Ile-Lys-Asn-Asn-Ser-Arg-Ser-Gly-Gly-Thr-Ile-Gly-Pro-Ser-Leu-Ala-Ser-Gln; and

l. Gly-Ser-Lys-Asp-Val-Gly-Leu-Gly-Val-Lys-Phe-Phe-Asn-Gly-Phe-Phe-Lys-His, from a sample containing said aminopeptidase comprising the following steps:

(a) recovering crude substrate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by said ion exchange chromatography, and optionally:

(d) subjecting said recovered substrate-specific aminopeptidase of step (c) to hydroxylapatite chromatography; and

(e) recovering any substrate-specific aminopeptidase purified by said hydroxylapatite chromatography, and, further optionally;

(f) subjecting said recovered substrate-specific aminopeptidase of step (e) to Phenyl-Sepharose CL-4B chromatography; and

(g) recovering any substrate-specific aminopeptidase purified by said Phenyl-Sepharose CL-4B chromatography.

3. A method for recovering the substantially purified substrate-specific aminopeptidase APX having an ability to cleave the peptide bond between an amino-terminal methionine residue, and every penultimate amino-terminal amino acid residue selected from the group consisting of Val, Ala, Arg, Asp, Cys, Glu, Gln, Gly, His, Ile, Leu, Lys, Met, Phe, Pro, Ser, Thr and Trp, and having an ability to cleave the peptide bond between an amino-terminal residue X, and an adjacent Ile residue of a peptide, wherein X is an amino acid residue selected from the group consisting of: Val, Ala, Arg, Gln, Gly, His, Ile, Leu, Met, Phe, Thr and Trp, from a sample containing said aminopeptidase comprising the following steps:

(a) recovering crude substate-specific aminopeptidase from a sample containing said aminopeptidase;

(b) subjecting said crude substrate-specific aminopeptidase from step (a) to ion exchange chromatography; and

(c) recovering any substrate-specific aminopeptidase purified by said ion exchange chromatography, and optionally:

(d) subjecting said recovered substrate-specific aminopeptidase of step (c) to hydroxylapatite

27

chromatography; and

(e) recovering any substrate-specific aminopeptidase purified by said hydroxylapatite chromatography.

4. A genetic engineering process which makes use of a vacuolar localization presequence comprising the polypeptide:
Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu.

5. The process of claim 4, wherein the vacuolar localization presequence additionally contains an amino-terminal methionine residue.

6. The process of claim 4 or 5, wherein the vacuolar localization presequence is linked via a peptide bond to the amino-terminus of a heterologous protein or polypeptide.

7. A process as claimed in claim 2, wherein the aminopeptidase API is linked to a vacuolar localization presequence selected from the group consisting of:

a.    Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu;
and

b.    Met-Glu-Glu-Gln-Arg-Glu-Ile-Leu-Glu-Gln-Leu-Lys-Lys-Thr-Leu-Gln-Met-Leu-Thr-Val-Glu-Pro-Ser-Lys-Asn-Asn-Gln-Ile-Ala-Asn-Glu-Glu-Lys-Glu-Lys-Lys-Glu-Asn-Glu-Asn-Ser-Trp-Cys-Ile-Leu.

8. A process for the preparation of the substrate-specific aminopeptidases, AP2, API or APX or a presequence thereof, which comprises expressing in a host cell a recombinant molecule encoding said enzyme or presequence.

9. A method for removing an amino-terminal methionine residue from a peptide containing said amino-terminal residue, and containing as a penultimate amino-terminal amino acid residue, an amino acid selected from the group consisting of Arg, Asp, Gln, Gly, His, Ile, Leu, Lys, Met, and Phe, which method comprises incubating said peptide in the presence of the substrate-specific aminopeptidase AP2 to thereby remove said amino-terminal residue.

10. A method for removing an amino-terminal methionine residue from a peptide containing said amino-terminal residue, and containing as a penultimate amino-terminal amino acid residue, an amino acid selected from the group consisting of Ala, Arg, Asp, Cys, Gly, His, Ile, Leu, Lys, Met, Phe, Ser and Trp, which method comprises incubating said peptide in the presence of the substrate-specific aminopeptidase API to thereby remove said amino-terminal residue.

11. A method for removing an amino-terminal methionine residue from a peptide containing said amino-terminal residue, and containing as a penultimate amino-terminal amino acid residue, an amino acid selected from the group consisting of Val, Ala, Arg, Asp, Cys, Glu, Gln, Gly, His, Ile, Leu, Lys, Met, Phe, Pro, Ser, Thr and Trp, which method comprises incubating said peptide in the presence of the substrate-specific aminopeptidase APX to thereby remove said amino-terminal residue.

12. A method for removing an amino-terminal residue X from a peptide containing said terminal residue X, and an adjacent Ile residue, wherein X is selected from the group consisting of Ala, Arg, Gln, His, Ile, Leu, Met, Phe, Pro, Ser, Thr and Trp, which method comprises incubating said peptide in the presence of the substrate-specific aminopeptidase AP2 to thereby remove said amino-terminal residue.

13. A method for removing an amino-terminal residue X from a peptide containing said terminal residue X, and an adjacent Ile residue, wherein X is selected from the group consisting of Arg, Gln, His, Leu, Met, Phe, Pro, Ser, Thr and Trp, which method comprises incubating said peptide in the presence of the substrate-specific aminopeptidase API to thereby remove said amino-terminal residue.

14. A method for removing an amino-terminal residue X from a peptide containing said terminal residue X, and an adjacent Ile residue, wherein X is selected from the group consisting of Val, Ala, Arg, Gln, Gly, His, Ile, Leu, Met, Phe, Thr and Trp, which method comprises incubating said peptide in the presence of the substrate-specific aminopeptidase APX to thereby remove said amino-terminal residue.

15. The method of any one of the claims 9 to 14, wherein said substrate-specific aminopeptidase is immobilized to a solid support.

$$a$$

H₂N-GLU-HIS-ASN-TYR-GLU-ASP-ILE-ALA-GLN-GLU-PHE-ILE·ASP-PHE-ILE-TYR-LYS-ASN·····

$$b$$

Probe  a : 5'- CA(C/T)AA(C/T)TA(C/T)GA(A/G)GA(C/T)AT(I)GC-3'

Probe  b : 5'- GA(A/G)GA(T/C)AT(I)GC(I)CA(A/G)GA(A/G)TT(T/C)AT- 3'

*FIG.1*

EP 0 359 164 A2

FIG.2

GTAAGTGAAATAGTTGACACACCCCTTAATCGTTCCATTAGATAATAAAAATTCATTTAT

AAGCAGTTGACTTCACAGAACAAGACATACACTGTATAGAGCCTGATCAGTAATCTTAGT

GCAATTGTAGAAACCTGCACAACCAACAAAATTAAGAAAAAAAGAATGGAGGAACAACGT
                                                MetGluGluGlnArg    -41

GAAATACTGGAACAATTGAAGAAAACTCTGCAGATGCTAACTGTAGAGCCATCTAAAAAT
GluIleLeuGluGlnLeuLysLysThrLeuGlnMetLeuThrValGluProSerLysAsn    -21

AACCAAATCGCCAACGAAGAAAAGGAAAAGAAAGAAAATGAAAATTCGTGGTGCATCCTC
AsnGlnIleAlaAsnGluGluLysGluLysLysGluAsnGluAsnSerTrpCysIleLeu    -1
                                                                           *

GAGCACAATTATGAGGATATTGCACAGGAATTCATTGATTTCATTTACAAGAACCCTACC
GluHisAsnTyrGluAspIleAlaGlnGluPheIleAspPheIleTyrLysAsnProThr    20

ACTTACCATGTAGTATCATTTTTCGCGGAGCTGTTAGATAAGCATAACTTCAAATACTTG
ThrTyrHisValValSerPhePheAlaGluLeuLeuAspLysHisAsnPheLysTyrLeu    40

AGCGAGAAATCCAATTGGCAGGACTCCATTGGCGAAGATGGTGGGAAATTCTACACTATA
SerGluLysSerAsnTrpGlnAspSerIleGlyGluAspGlyGlyLysPheTyrThrIle    60
               *             *

AGAAATGGAACTAACCTATCTGCCTTTATCCTGGGCAAAAACTGGAGAGCCGAAAAGGGT
ArgAsnGlyThrAsnLeuSerAlaPheIleLeuGlyLysAsnTrpArgAlaGluLysGly    80

GTCGGTGTCATTGGATCTCATGTGGACGCTTTGACGGTCAAATTGAAGCCCGTCTCCTTT
ValGlyValIleGlySerHisValAspAlaLeuThrValLysLeuLysProValSerPhe    100

AAAGACACAGCGGAAGGTTACGGAAGAATTGCTGTTGCTCCCTATGGAGGTACACTGAAT
LysAspThrAlaGluGlyTyrGlyArgIleAlaValAlaProTyrGlyGlyThrLeuAsn    120

GAATTGTGGCTAGACAGAGACCTAGGTATTGGTGGTCGCCTTCTTTACAAGAAGAAGGGC
GluLeuTrpLeuAspArgAspLeuGlyIleGlyGlyArgLeuLeuTyrLysLysLysGly    140


# FIG. 3A

ACTAACGAAATTAAAAGCGCCTTGGTTGATTCTACACCCCTACCTGTTTGTCGAATTCCT
ThrAsnGluIleLysSerAlaLeuValAspSerThrProLeuProValCysArgIlePro    160

TCCTTGGCTCCCCATTTCGGTAAACCTGCTGAAGGCCCATTTGATAAAGAGGACCAAACT
SerLeuAlaProHisPheGlyLysProAlaGluGlyProPheAspLysGluAspGlnThr    180

ATCCCGGTCATCGGCTTCCCCACTCCGGATGAGGAAGGTAATGAACCTCCCACGGATGAT
IleProValIleGlyPheProThrProAspGluGluGlyAsnGluProProThrAspAsp    200

GAAAAGAAATCGCCCTTATTTGGCAAACACTGCATCCACCTGTTAAGGTACGTTGCCAAA
GluLysLysSerProLeuPheGlyLysHisCysIleHisLeuLeuArgTyrValAlaLys    220

TTAGCCGGTGTGGAAGTGTCCGAATTGATTCAAATGGATTTAGACTTATTCGATGTGCAA
LeuAlaGlyValGluValSerGluLeuIleGlnMetAspLeuAspLeuPheAspValGln    240

AAGGGTACCATTGGAGGTATTGGTAAACACTTCCTTTTTGCACCACGTCTAGATGACAGG
LysGlyThrIleGlyGlyIleGlyLysHisPheLeuPheAlaProArgLeuAspAspArg    260

TTGTGTAGTTTCGCAGCAATGATTGCTTTGATTTGCTACGCTAAGGATGTTAATACCGAG
LeuCysSerPheAlaAlaMetIleAlaLeuIleCysTyrAlaLysAspValAsnThrGlu    280

GAATCAGATTTATTCTCTACTGTCACTTTGTATGATAATGAAGAAATCGGATCGTTGACA
GluSerAspLeuPheSerThrValThrLeuTyrAspAsnGluGluIleGlySerLeuThr    300

AGACAAGGCGCAAAAGGTGGCTTGTTGGAGTCAGTGGTGGAACGCAGTTCTTCTGCATTC
ArgGlnGlyAlaLysGlyGlyLeuLeuGluSerValValGluArgSerSerSerAlaPhe    320

ACTAAGAAACCGGTCGATTTGCATACGGTTTGGGCTAATTCCATCATCTTGTCCGCAGAC
ThrLysLysProValAspLeuHisThrValTrpAlaAsnSerIleIleLeuSerAlaAsp    340

GTCAACCACCTCTACAACCCAAACTTTCCTGAAGTCTATTTGAAGAATCATTTTCCAGTG
ValAsnHisLeuTyrAsnProAsnPheProGluValTyrLeuLysAsnHisPheProVal    360

# FIG. 3B

CCTAATGTCGGAATCACTTTATCACTGGATCCTAACGGTCATATGGCCACAGATGTCGTA
ProAsnValGlyIleThrLeuSerLeuAspProAsnGlyHisMetAlaThrAspValVal 380

GGAACTGCCCTAGTAGAAGAATTAGCACGCCGCAATGGAGACAAAGTGCAATATTTCCAA
GlyThrAlaLeuValGluGluLeuAlaArgArgAsnGlyAspLysValGlnTyrPheGln 400
          *
ATCAAAAACAACTCAAGATCAGGTGGTACTATCGGCCCATCATTGGCTTCTCAAACAGGT
IleLysAsnAsnSerArgSerGlyGlyThrIleGlyProSerLeuAlaSerGlnThrGly 420

GCTCGTACCATAGACCTGGGAATTGCACAGTTGTCCATGCACAGCATCAGAGCTGCTACA
AlaArgThrIleAspLeuGlyIleAlaGlnLeuSerMetHisSerIleArgAlaAlaThr 440

GGGTCCAAGGATGTCGGATTAGGTGTTAAGTTCTTCAACGGATTTTTCAAGCACTGGAGA
GlySerLysAspValGlyLeuGlyValLysPhePheAsnGlyPhePheLysHisTrpArg 460

TCAGTCTACGATGAATTCGGCGAGTTGTGATTTTGCCACACTCTTTTTATTTCTTTTGAT
SerValTyrAspGluPheGlyGluLeuEnd

TTCTGTTTCTTTATCCTTTTTCTAAAAATACTTTTATTTCGAATCAGGAAGAAAAGAAAA

ATGTATAAAAAAAAAAAAAAATAAAACACCACAATCTCGTCAAGATATAAATATCTATTTAT

AAATAAACATCAAAATACCTAAACCCCAACA

# FIG.3C

FIG. 4